(19) Europäisches Patentamt
European Patent Office
Office européen des brevets

(11) Numéro de publication: **0 024 960**
**B1**

(12) **FASCICULE DE BREVET EUROPEEN**

(45) Date de publication du fascicule du brevet: **12.12.84**

(21) Numéro de dépôt: **80400999.1**

(22) Date de dépôt: **01.07.80**

(51) Int. Cl.³: **C 07 D 207/08,**
C 07 D 223/08, A 61 K 31/40,
A 61 K 31/55

(54) **Nouvelles phénoxyamines hétérocycliques substituées, procédé pour leur préparation et leur application comme anesthésiques locaux.**

(30) Priorité: **06.07.79 FR 7917610**
**01.04.80 FR 8007352**

(43) Date de publication de la demande:
**11.03.81 Bulletin 81/10**

(45) Mention de la délivrance du brevet:
**12.12.84 Bulletin 84/50**

(84) Etats contractants désignés:
**AT BE CH DE FR GB IT LI LU NL SE**

(56) Documents cités:
**EP-A-0 002 672**
**EP-A-0 004 288**
**DE-A-2 026 688**
**DE-C- 543 876**
**FR-A-2 155 927**
**FR-B-1 342 756**
**GB-A-1 096 441**

**Le dossier contient des informations techniques présentées postérieurement au dépôt de la demande et ne figurant pas dans le présent fascicule.**

(73) Titulaire: **SOCIETE D'ETUDES SCIENTIFIQUES ET INDUSTRIELLES DE L'ILE DE FRANCE**
**46, Boulevard de Latour-Maubourg**
**F-75340 Paris Cedex 07 (FR)**

(72) Inventeur: **Thominet, Michel**
**82, rue Bonaparte**
**F-75006 Paris (FR)**
Inventeur: **Franceschini, Jacqueline**
**28, Avenue Larroumes**
**F-94240 L'Hay-les-Roses (FR)**

(74) Mandataire: **Gallochat, Alain**
**SOCIETE D'ETUDES SCIENTIFIQUES ET INDUSTRIELLES DE L'ILE DE FRANCE 46, Boulevard de Latour Maubourg**
**F-75340 Paris Cedex 07 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 024 960 B1

**Description**

La présente invention a pour objet de nouvelles phénoxyamines hétérocycliques substituées de formule générale 1, leurs sels d'addition avec les acides pharmaceutiquement acceptables, leurs sels d'ammonium quaternaire, leurs N-oxydes ainsi que les méthodes de préparation de ces composés et les médicaments qui en renferment en tant que principes actifs.

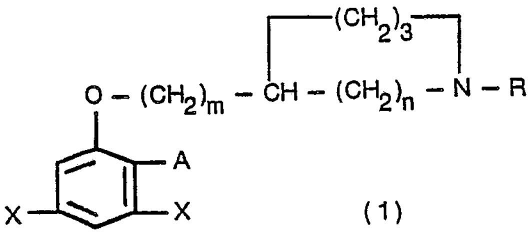

(1)

Dans cette formule m = 0 ou 2
n = 0 ou 2
avec la condition m + n = 2
et dans laquelle:
A = — hydrogène,
— alcoxy comportant 1 à 4 atomes de carbone, tel que méthoxy ou éthoxy, propoxy ou butoxy linéaire ou ramifié,
— alcényloxy comportant 2 à 6 atomes de carbone, tel que vinyloxy, propènyloxy (allyloxy), butènyloxy, pentènyloxy, hexènyloxy,
X = — halogène tel que F, Cl, Br
R = — hydrogène,
— alkyle inférieur comportant 1 à 6 atomes de carbone tel que méthyle ou éthyle, propyle, butyle, pentyle ou hexyle linéaire ou ramifié,
— cycloalkyle comportant au moins 3 atomes de carbone et pouvant être substitué par un groupe alkyle inférieur, tel que cyclopropyle, cyclobutyle, cyclopentyle, cyclohexyle, méthylcyclobutyle, méthylcyclopentyle, méthylcyclohexyle, éthylcyclohexyle,
— alcènyle comportant 2 à 6 atomes de carbone, tel que vinyle, propèn-2-yle (allyle), butènyle, pentènyle, hexènyle,
— cycloalcènyle comportant en particulier plus de 3 atomes de carbone et pouvant être substitué par un groupe alkyle inférieur, tel que cyclobutènyle, cyclopentènyle, cyclohexènyle, méthylcyclobutènyle, méthylcyclopentènyle, méthylcyclohexènyle, éthylcyclohexènyle,
— cycloalkyl- ou cycloalcènyl-alkyle où les groupes cycloalkyle, cycloalcènyle et alkyle sont définis comme précédemment, tel que cyclopropylméthyle, cyclopropyléthyle, cyclobutylméthyle, cyclobutyléthyle, cyclopentylméthyle, cyclopentyléthyle, cyclopentènylméthyle, cyclopentènyléthyle, cyclohexylméthyle, cyclohexyléthyle, cyclohexènylméthyle, cyclohexènyléthyle.

Les composés de l'invention peuvent être représentés par les formules générales (II) et (III) suivantes, les composés de formule (III) étant les composés préférentiels:

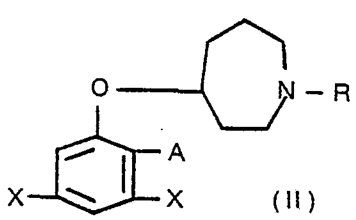

(II)

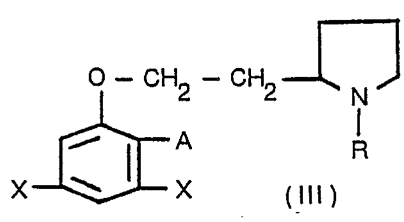

(III)

dans lesquelles R, A et X sont définis comme précédemment.

Les composés particulièrement intéressants sont les composés de formule (IV):

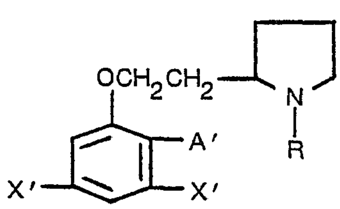

(IV)

dans laquelle:

A′ représente un atome d'hydrogène, un groupe méthoxy ou éthoxy

X′ représente un atome de chlore ou de brome

R est défini comme précédemment.

L'invention s'étend aussi aux formes optiquement actives des composés de formule générale 1 ainsi qu'à leurs sels d'addition avec les acides pharmaceutiquement acceptables, leurs sels d'ammonium quaternaire, leurs N-oxydes.

Par suite de l'existence dans ces composés d'un carbone asymétrique ceux-ci peuvent se présenter sous forme racémique ou sous forme optiquement active après dédoublement.

Les produits de l'invention manifestent des propriétés pharmacologiques intéressantes sur le système nerveux central notamment comme anesthésiques locaux.

L'invention s'étend donc à l'application comme médicament des composés de formule générale 1.

L'invention comprend encore les compositions pharmaceutiques renfermant comme principe actif au moins un des composés de formule générale 1 associé à un excipient inerte pharmaceutique.

L'invention concerne également un procédé d'obtention des composés de formule générale 1 caractérisé en ce que l'on condense un phénol répondant à la formule général V:

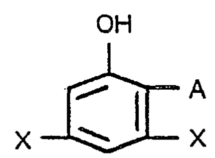

(V)

dans laquelle A et X sont définis comme ci-dessus, avec un composé de formule générale VI:

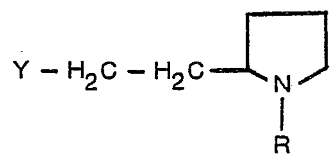

(VI)

dans laquelle R a les significations déjà données et Y est un reste anionique susceptible d'être éliminé, le reste Y sera par exemple un atome d'halogène, en particulier le chlore, le brome ou l'iode, pour obtenir un composé de formule générale 1:

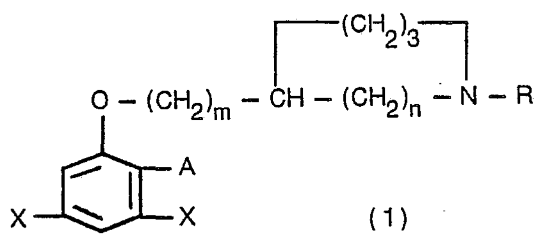

(1)

dans laquelle la définition de m, n, A, X et R demeure inchangée. Ces composés peuvent être salifiés. Ils peuvent être dédoublés par réaction avec un acide optiquement actif en leurs isomères optiques actifs pharmacologiquement.

Les phénols de formule V sont mis en jeu sous forme de phénolates de métaux alcalins particulièrement de phénolate de sodium que l'on obtient par exemple par réaction des phénols avec les alcoolates de métaux alcalins. On effectue la réaction des composés de formule V avec les composés de formule VI dans un solvant organique inerte comme par exemple le toluène, le xylène .... On opère à la température de reflux du mélange réactionnel. On obtient des composés qui sont ensuite séparés, isolés et purifiés selon les méthodes habituelles, par exemple, extraction, formation de sels, recristallisation, chromatographie, etC ...

La salification des composés de formule générale 1 est effectuée de préférence par addition d'un acide minéral comme par exemple l'acide chlorhydrique, l'acide bromhydrique, l'acide phosphorique ou d'un acide organique comme par exemple l'acide fumarique, l'acide citrique, l'acide oxalique.

Les composés de formule générale 1 peuvent également réagir par exemple, avec des halogénures ou des sulfates d'alkyle pour donner des sels d'ammonium quaternaires.

Les composés de formule 1 peuvent être oxydes d'une manière connue en soi par exemple au moyen d'eau oxygénée et de bioxyde de manganèse pour donner les N-oxydes correspondants.

Le dédoublement des composés de formule générale 1 est effectué avec un acide optiquement actif.

Les composés de départ de formule V dans laquelle X représente le chlore peuvent être préparés à

partir de phénols par acétylation, chloration, désacétylation puis purification.

Les composés de départ de formule V dans laquelle X représente le brome peuvent être préparés à partir de l'o.nitrophénol par bromuration, alkylation de la fonction phénol, réduction du groupe nitro, diazotation et décomposition.

Les composés de départ de formule VI peuvent être préparés selon la 4ème méthode décrite par YAO-HUA-WU et J. R. CORRIGAN — J. ORG. CHEM. (1961) p. 1531.

Afin d'illustrer les caractéristiques techniques de la présente invention, quelques exemples de réalisation vont être décrits étant bien entendu que ceux-ci ne sont pas limitatifs quant à leur mode de mise en oeuvre et aux applications que l'on peut en faire.

Exemple I

1-méthyl-4-[2-méthoxy-3,5-dichlorophénoxy]hexaméthylène imine 1-méthyl-2-[2-méthoxy-3,5-dichloro phénoxyéthyl]pyrrolidine

Dans un ballon de deux litres muni d'un agitateur étanche, d'un réfrigérant à reflux et d'un thermomètre on dissout 13 g de sodium dans 180 ml d'éthanol et on ajoute à la solution obtenue 108 g de 3,5-dichloro gaïacol (0,56 mole) et 300 ml de toluène sec.

Le réfrigérant est alors remplacé par une colonne de Vigreux de 40 cm et la totalité de l'alcool est éliminée par distillation azéotropique. Au fur et à mesure de l'élimination de l'alcool le sel de sodium du 3,5-dichloro gaïcol cristallise en une masse épaisse.

En fin de distillation on ajoute un volume de toluène égal à celui qui a été entraîné puis on refroidit.

La colonne de Vigreux est remplacée de nouveau par le réfrigérant à reflux et 103 g de 1-méthyl-2-[2-chloroéthyl]pyrrolidine (0,56 mole + 25% d'excès) sont ajoutés. Le mélange obtenu est porté doucement au reflux. Rapidement le mélange se fluidifie avec un net dégagement de chaleur.

Dès que la réaction est calmée on chauffe de nouveau au reflux et on le maintient 8 heures.

Le mélange réactionnel est alors refroidi et repris par 400 ml d'eau et 80 ml d'acide chlorhydrique concentré. Le toluène est décanté et lavé 2 fois à l'eau acide.

Les solutions aqueuses sont réunies, filtrées avec du noir et alcalinisées par addition d'ammoniaque à 20% jusqu'à virage de la phénolphtaléine.

L'huile qui se sépare est décantée et extraite à l'éther. La solution éthérée est séchée sur carbonate de potassium.

Puis l'éther est distillé en terminant sous vide jusqu'à poids constant.

On obtient 182 g de produit (théorie: 170 g)

D'après l'analyse C.C.M. (Silice MERCK 5554 — Eluant = benzène, éthanol, ammoniaque (84:15:1), le produit obtenu est un mélange en parties sensiblement égales de deux isomères:

1-méthyl-4-[2-méthoxy-3,5-dichlorophénoxy]hexaméthylène imine et 1-méthyl-2-[2-méthoxy-3,5-dichlorophénoxyéthyl]pyrrolidine.

Le mélange de bases est dissous dans 400 ml de méthyl éthyl cétone et l'on ajoute 20,5 g d'acide chlorhydrique (0,56 mole) sec en solution dans 40 ml de méthyl éthyl cétone.

La cristallisation est amorcée et abandonnée une nuit. Le chlorhydrate est alors essoré, lavé avec de la méthyl éthyl cétone et séché à 40°.

On obtient 97 g de produit. P.F. = 155—7°C.

Ce chlorhydrate est constitué en majorité, d'après l'analyse C.C.M. de dérivé hexaméthylène imine.

Les eaux mères sont traitées ultérieurement pour l'obtention du dérivé pyrrolidinique.

Le chlorhydrate est recristallisé deux fois dans respectivement 185 et 150 ml d'acétronitrile. On recueille 69,5 g de chlorhydrate de 1-méthyl-4-[2-méthoxy-3,5-dichlorophénoxy]hexaméthylène imine fondant à 161—162°5C.

Les eaux mères du chlorhydrate sont reprises avec un peu d'eau et la méthyl éthyl cétone est distillée. La solution restante est diluée avec 325 ml d'eau, filtrée avec du noir et alcalinisée par addition d'ammoniaque à 20% jusqu'à virage de la phénolphtaléine. L'huile qui se sépare est décantée et extraite à l'éther. La solution éthérée est séchée sur carbonate de potassium puis l'éther est distillé en terminant sous vide jusqu'à poids constant. Poids obtenu = 82 g.

Par dissolution à chaud dans 230 ml d'isopropanol des 82 g de base (0,27 mole) et de 31,5 g d'acide fumarique (0,27 mole) puis refroidissement et filtration du précipité formé on obtient 109,5 g de fumarate.

Celui-ci est recristallisé dans 275 ml de méthanol. On obtient 78 g de fumarate de 1-méthyl-2-[2-méthoxy-3,5-dichlorophénoxyéthyl]pyrrolidine fondant à 179—180°C.

L'analyse C.C.M. révèle la présence d'une faible quantité de dérivé hexaméthylène imino non décelée par le spectre RMN.

Exemple II

1-méthyl-2-[2-méthoxy-3,5-dichlorophénoxyéthyl]pyrrolidine dextrogyre

Le fumarate du produit racémique est transformé en base par alcalinisation à l'ammoniaque aqueuse et extraction à l'éther. 161 g de cette base (0,53 mole) sont dissous dans 320 ml de méthanol et une solution de 199 g d'acide dibenzoyltartrique L (+) (0,53 mole) dans 400 ml de méthanol est ajoutée. Le dibenzoyltartrate cristallise immédiatement. Après une nuit de repos il est assoré, lavé avec 300 ml de méthanol et séché à 40°C. On obtient 161 g de produit fondant à 160°C $[\alpha]_D^{20} = -37°$ (solution 5% dimethylformamide).

157 g de dibenzoyltartrate sont dissous dans 200 ml de diméthylformamide puis 65 ml d'eau sont ajoutés (ce qui donne du diméthylformamide à 88%).

Après refroidissement le sel qui recristallise est essoré, lavé avec 200 ml de diméthylformamide à 80% puis à l'eau et séché à 40°. On recueille 136 g de produit. P.F. = 140—1°C.

$[\alpha]_D^{20} = -36°,5$ (solution 5% diméthylformamide).

Dans un ballon de trois litres muni d'un agitateur on introduit 136 g de dibenzoyltartrate, 600 ml d'eau 45 ml d'ammoniaque à 20% et 300 ml d'éther. La base qui précipite se dissout aussitôt dans l'éther.

Elle est décantée. La solution aqueuse est extraite à l'éther.

La solution éthérée ainsi obtenue est séchée sur carbonate de potassium puis l'éther est distillé en terminant sous vide jusqu'à poids constant = 61,5 g.

$[\alpha]_D^{20} = +47°,5$ (solution 5% dimethylformamide).

Ces 61,5 g de base (0,202 mole) sont chauffés en présence de 170 ml d'eau et 23,5 g d'acide fumarique (0,202 mole) jusqu'à dissolution. La solution bouillante obtenue est filtrée avec du noir. Par refroidissement le fumarate cristallise lentement. Il est essoré, lavée à l'eau et séché à 40°C.

On obtient 74 g de fumarate fondant à 157—7°5C.

$[\alpha]_D^{20} = +19°,5$ (solution 5% diméthylformamide).

Exemple III

1-méthyl-2-[2-méthoxy-3,5-dichlorophénoxyéthyl]pyrrolidine lévogyre

Les jus alcooliques provenant de la préparation du dibenzoyltartrate de 1-méthyl-2-[2-méthoxy-3,5-dichlorophénoxyéthyl]pyrrolidine dextrogyre et contenant environ 88 g de base sont concentrés. Le résidu est repris par 400 ml d'eau, 60 ml d'ammoniaque à 20% et de l'éther en agitant vigoureusement.

La base qui précipite se dissout dans l'éther. La solution éthérée est décantée. La phase aqueuse est extraite 3 fois à l'éther.

Les phases éthérées sont réunies et séchées sur $K_2CO_3$. L'éther est distillé en terminant sous vide jusqu'à poids constant. On obtient 69,5 g de base.

67 g de cette base (0,22 mole) sont dissous dans 140 ml de méthanol puis l'on ajoute une solution de 83 g d'acide dibenzoyltartrique D (—) (0,22 mole) dans 165 ml de méthanol. Le dibenzoyltartrate cristallise immédiatement. Il est essoré, lavé au méthanol, séché à 40°C.

Poids obtenu: 126 g. P.F. = 133—4°C.

$[\alpha]_D^{20} = +37°2$ (solution 5% diméthylformamide).

124 g de dibenzoyltartrate sont repris dans l'eau et un excès d'ammoniaque. La base qui se sépare est aussitôt extraite à l'éther.

La solution éthérée est décantée. La phase aqueuse est extraite à l'éther. Les phases éthérées sont réunies et séchées sur carbonate de potassium. L'éther est ensuite distillé en terminant sous vide jusqu'à poids constant: 56 g

$[\alpha]_D^{20} = -44°8$

Dans un ballon de 500 ml muni d'un réfrigérant à reflux on introduit 54 g de base (0,178 mole) 145 ml d'eau et 21 g d'acide fumarique (0,178 mole) et on chauffe jusqu'à dissolution. La solution bouillante obtenue est filtrée avec du noir. Par refroidissement le fumarate cristallise. Il est essoré, lavé à l'eau et séché à 40°.

On obtient 68 g de produit fondant à 157—8°C.

$[\alpha]_D^{20} = -17°8$ (solution 5% diméthylformamide).

Exemple IV

1-allyl-4-[2-méthoxy 3,5-dichlorophénoxy]hexaméthylène imine

1-allyl-2-[2-méthoxy 3,5-dichlorophénoxyéthyl]pyrrolidine

En suivant le mode opératoire de la réaction du 3,5-dichloro gaïacol avec la 1-méthyl-2-[2-chloroéthyl]pyrrolidine on obtient à partir de 246 g de 3,5-dichloro gaïacol (1,275 moles) et 221 g de 1-allyl-2-[2-chloroéthyl]pyrrolidine (1,275 moles), 411 g d'un mélange en parties sensiblement égales de deux isomères:

la 1-allyl-4-[2-méthoxy 3,5-dichlorophénoxy]hexaméthylène imine et la 1-allyl-2-[2-méthoxy 3,5-dichlorophénoxyéthyl]pyrrolidine.

397 g du mélange de bases (1,20 moles) sont dissous dans 1250 ml d'acétonitrile puis l'on ajoute 230 g d'acide citrique sec (1,20 moles). On chauffe la suspension jusqu'à dissolution totale, puis glace la solution pendant une nuit. On essore le précipité, le lave avec 1200 ml d'acétronitrile, le sèche à l'air puis en étuve à 40°c. On obtient 538 g d'un mélange de citrates des deux produits. Ce mélange est recristallisé 3 fois avec passage au noir dans l'alcool à 95°. On obtient 227 g de produit dont le spectre RMN est compatible avec la structure hexaméthylène imine P.F. = 90—5°C.

L'acétonitrile et les jus alcooliques de recristallisation sont distillés en terminant sous vide. le

résidu est repris à l'eau et filtré avec du noir. La base est ensuite précipitée par addition d'ammoniaque à 20% jusqu'à virage de la phénolphtaléine. L'huile qui se sépare est décantée et extraite à l'éther. La solution éthérée est séchée sur carbonate de potassium et l'éther est distillé en terminant sous vide jusqu'à poids constant. On obtient 184 g de base (0,56 mole) que l'on dissout à chaud dans 550 ml d'isopropanol et 65 g d'acide fumarique (0,56 mole). Par refroidissement le fumarate cristallise, il est essoré, lavé à l'isopropanol, séché à l'air puis à 40°C. On obtient 141 g de produit fondant à 135—6°.

140 g de fumarate sont recristallisés dans 275 ml puis 145 ml d'isopropanol et finalement dans 190 ml d'eau. On obtient 90 g de fumarate de 1-allyl-2-[2-méthoxy 3,5-dichlorophénoxyéthyl]pyrrolidine. P.F. = 37—8°C

## Exemple V

1-éthyl-2-[3,5-dichlorophénoxyéthyl]pyrrolidine

Dans un ballon de deux litres muni d'un agitateur étanche, d'un réfrigérant à reflux et d'un thermomètre on dissout 19 g de sodium dans 245 ml d'alcool absolu. On refroidit la solution d'éthylate et lui ajoute 133 g de dichlorophénol (0,815 mole) et 430 ml de toluène sec. Le réfrigérant à reflux est remplacé par une colonne de Vigreux et l'alcool est éliminé par distillation azéotropique. Le sel de sodium du dichlorophénol précipite et fait épaissir le milieu qui reste cependant agitable.

Après refroidissement on remplace la colonne de Vigreux par un réfrigérant à reflux, on ajoute 139 g de 1-éthyl-2-[β-chloroéthyl]pyrrolidine (0,815 mole + 5%) et on chauffe au reflux 8 heures. La 1-éthyl-2-[β-chloroéthyl]pyrrolidine a été préparée à partir de son chlorhydrate immédiatement avant son utilisation.

La réaction terminée le mélange réactionnel est repris par 1,8 litres d'eau et 85 ml d'acide chlorhydrique concentré. La phase toluénique est décantée puis lavée avec 100 ml d'eau et 10 ml d'acide chlorhydrique concentré.

Les phases aqueuses sont réunies, filtrées avec du noir et alcalinisées par addition d'ammoniaque à 20% jusqu'à virage de la phénolphtaléine. L'huile qui se sépare est extraite à l'éther. La solution éthérée est séchée sur carbonate de potassium. Après élimination de l'éther, le produit est distillé sous vide. Coeur 180—2°C sous 5 mmHg (665 Pa). Poids = 175 g.

Ces 175 g de base (0,61 mole) sont dissous dans 335 ml de méthyl éthyl cétone puis l'on ajoute une solution de 22,5 g d'acide chlorhydrique sec (0,61 mole) dans 260 ml de méthyl éthyl cétone jusqu'à virage du rouge de méthyle. Le chlorhydrate cristallise lentement. Il est essoré, lavé avec 130 ml de méthyl éthyl cétone et séché à l'étuve. On obtient 151,5 g de chlorhydrate.

L'analyse chromatographie de ce produit (Silice Merck 5554—Eluant: Benzène-éthanol-ammoniaque 84:15:1) indique qu'il s'agit d'un mélange des chlorhydrates de 1-éthyl-2-[3,5-dichlorophénoxyéthyl]pyrrolidine et de 1-éthyl-4-[3,5-dichlorophénoxy]hexaméthylène imine. Le mélange est plus riche en pyrrolidine.

150 g du mélange des chlorhydrates sont recristallisés dans 150 ml puis dans 225 ml d'acétonitrile. On recueille 46 g de produit fondant à 138—140°C. Ils sont encore recristallisés dans 95 ml puis 50 ml d'isopropanol. On obtient 27 g de produit fondant à 152—3°C. L'analyse chromatographie ne présente qu'une seule tache.

## Exemple VI

1-méthyl-2-[2-méthoxy-3,5-dibromophénoxyéthyl]pyrrolidine

— *3,5-dibromo gaïacol*

a) Diazotation:

Dans un ballon de 3 litres muni d'un agitateur, d'un thermomètre et d'une ampoule à brome on introduit 725 ml d'acide sulfurique concentré et ajoute peu à peu 177 g de 3,5-dibromo o.anisidine (0,63 mole). La température monte jusqu'à 40°C. On refroidit la solution obtenue puis coule goutte à goutte entre 0° et +5°C une solution de 52,5 g de nitrite de sodium (0,63 mole + 20% excès) dans 80 ml d'eau.

La diazotation terminée on agite encore pendant 2 heures entre 0° et 5°C puis on verse la solution sur 1200 g de glace: on obtient une suspension.

b) Décomposition:

Dans un ballon de Vigreux de deux litres on dissout 120 g de sulfate de cuivre dans 1400 ml d'eau, porte à ébullition et ajoute goutte à goutte le diazoïque. Il se décompose et le phénol formé est entraîné à la vapeur. L'introduction terminée on continue l'entrainement. On distille 12 litres d'eau. Le phénol est décanté et la solution aqueuse extraite à l'éther.

La phase organique est reprise à la soude diluée.

On décante puis acidifie la phase aqueuse par l'acide chlorhydrique. Le phénol reprécipite. Il est extrait à l'éther, la solution éthérée est séchée sur $Na_2SO_4$ puis l'éther est distillé en terminant sous vide jusqu'à poids constant. Le résidu est repris par deux volumes d'éther de pétrole. Le phénol se dissout puis cristallise en refroidissant. Il est essoré, lavé à l'éther de pétrole et séché à l'air. Poids: 54 g. P.F. = 59—60°C. Après recristallisation dans l'éther de pétrole on obtient 43 g de 3,5-dibromo gaïacol fondant à 63—4°C.

— *1-méthyl-2-[2-méthoxy-3,5-dibromophénoxyéthyl]pyrrolidine*

Dans un ballon de deux litres muni d'un agitateur, d'un réfrigérant à reflux et d'un thermomètre on dissout 15 g de sodium dans 215 ml d'éthanol absolu. On ajoute 187 g de dibromo gaïacol (0,66 mole) dans 365 ml de toluène sec. On élimine la totalité de l'alcool par distillation azéotropique. On obtient une suspension très épaisse à laquelle on rajoute 190 ml de toluène. On refroidit puis ajoute 102,5 g de 1-méthyl-2-[β-chloroéthyl pyrrolidine (0,66 mole + 5% d'excès). On chauffe 8 heures à reflux.

Puis on reprend le mélange réactionnel par 500 ml d'eau et 66 ml d'acide chlorhydrique (d = 1,18).

On décante la couche toluénique et la lave avec 200 ml d'acide chlorhydrique dilué 1/20.

On réunit les solutions aqueuses, les filtre avec du noir puis alcalinise à l'ammoniaque à 20%. L'huile qui décante est extraite à l'éther.

La solution éthérée est séchée sur $K_2CO_3$ puis l'éther est distillé jusqu'à poids constant.

On obtient 165 g de base qui d'après l'analyse C.C.M. est un mélange d'isomères:

1-méthyl-4-[2-méthoxy-3,5-dibromophénoxy]héxaméthylène imine et 1-méthyl-2-[2-méthoxy-3,5-dibromophénoxyéthyl]pyrrolidine.

164 g de base (0,448 mole) sont dissous dans de l'éthanol et l'on ajoute une solution de 52 g d'acide fumarique (0,448 mole) dans 685 ml d'éthanol absolu. Par refroidissement le fumarate cristallise. Il est essoré, lavé à l'alcool, séché. L'analyse par C.C.M. de ce produit indique un enrichissement en dérivé pyrrolidinique. Le fumarate est purifié par une recristallisation dans 320 ml de méthanol suivie de deux recristallisations dans respectivement 100 ml et 80 ml de diméthylformamide. On obtient 33 g de fumarate de 1-méthyl-2-[2-méthoxy-3,5-dibromophénoxyéthyl]pyrrolidine fondant à 192°C.

## Exemple VII

1-éthyl-2-[2-méthoxy-3,5-dichlorophénoxyéthyl]pyrrolidine

Suivant le mode opératoire des exemples précédents en mettant en réaction 333 g de gaïacol (1,72 moles) et 292 g de 1-éthyl-2-[β-chloroéthyl]pyrrolidine (1,72 moles + 5% excès) préparée extemporanément à partir de son chlorhydrate on obtient après distillation 414 g de produit qui passent à 173—180°C sous 1 mmHg (133 Pa). Ce produit est dissous dans 800 ml de méthyl éthyl cétone. On ajoute une solution de 47,5 g d'acide chlorhydrique sec dans 400 ml de méthyl-éthyl-cétone. Le chlorhydrate qui cristallise est refroidi puis essoré, lavé à la méthyl-éthyl-cétone et séché en étuve à 40°C. On obtient 306,5 g de chlorhydrate fondant à 126—8°C que l'on recristallise dans 613 ml d'acétone.

On recueille 252 g de chlorhydrate de 1-éthyl-2-[2-méthoxy-3,5-dichlorophénoxyéthyl]pyrrolidine. P.F. = 129—130°C.

## Exemple VIII

1-éthyl-2-[2-méthoxy-3,5-dichlorophénoxyéthyl]pyrrolidine dextrogyre

175 g de 1-éthyl-2-[2-méthoxy-3,5-dichlorophénoxyéthyl]pyrrolidine (0,55 mole) sont dissous dans 260 ml d'éthanol à 95°. On ajoute une solution de 82,5 g d'acide tartrique dextrogyre (0,55 mole) dans 260 ml d'éthanol à 95°. Après refroidissement et amorçage le tartrate cristallise. Il est essoré, lavé avec 100 ml d'éthanol à 95° et séché à 40°C. On obtient 104 g de tartrate dextrogyre.

$[\alpha]_D^{20} = +21°5$ (solution aqueuse 5%)

103,5 g de tartrate sont recristallisés dans 207 ml d'éthanol à 95°. On récupère 82 g de produit.

$[\alpha]_D^{20} = +24°3$ (solution aqueuse 5%)

81 g de tartrate sont dissous dans 425 ml d'eau tiède puis la base est précipitée par addition d'ammoniaque à 20%. L'huile qui décante est extraite à l'éther. Après séchage de la phase éthérée et évaporation on obtient 47,5 g de base.

$[\alpha]_D^{20} = +55°8$ (solution 5% diméthylformamide)

46 g de base (0,145 mole) sont dissous dans 140 ml d'acétate d'éthyle et l'on ajoute une solution de 5,5 g d'acide chlorhydrique sec (0,145 mole) dans 55 ml d'acétate d'éthyle.

Le chlorhydrate qui cristallise est essoré, lavé à l'acétate d'éthyle et séché en étuve à 40°C. On obtient 47,5 g de produit fondant à 121—2°C.

$[\alpha]_D^{20} = +18°9$ (solution aqueuse 5%)

## Exemple IX

1-éthyl-2-[2-méthoxy-3,5-dichlorophénoxyéthyl]pyrrolidine lévogyre

Les jus alcooliques provenant d'une part de la précipitation d'autre part de la recristallisation du tartrate dextrogyre de 1-éthyl-2-[2-méthoxy-3,5-dichlorophénoxyéthyl]pyrrolidine sont repris par 850 ml d'eau et concentrés jusqu'à un volume de 400 ml.

On ajoute une solution de 31 g de chlorure de potassium (0,375 mole + 10% d'excès) dans 140 ml d'eau. Le tartrate de potassium qui précipite est essoré, lavé à l'eau.

Les jus sont alcalinisés à l'ammoniaque à 20%. L'huile qui se sépare est extraite à l'éther. La solution éthérée est séchée sur carbonate de potassium. L'éther est ensuite distillé en terminant sous vide jusqu'à poids constant. On obtient 107 g de produit qui est un mélange d'environ 20% de base dextrogyre et 80% de base lévogyre.

On dissout ce produit dans 160 ml d'éthanol absolu puis ajoute 53 g d'acide tartrique lévogyre dissous à chaud dans 160 ml d'éthanol. On filtre la solution puis la refroidit. Le tartrate qui cristallise est essoré, lavé à l'éthanol à 95° et séché en étuve à 40°C. Poids obtenu: 107,5 g

$[\alpha]_D^{20} = -21°6$ (solution aqueuse 5%)

Le tartrate est recristallisé dans 215 ml d'éthanol à 95°. On recueille 95 g de produit qui fond vers 80—85° puis cristallise et fond à 102—3°C. Il contient une mole d'eau.

$[\alpha]_D^{20} = -24°7$ (solution aqueuse 5%)

94 g de tartrate sont dissous dans l'eau tiède.

Le base est précipitée par addition d'ammoniaque à 20%, puis extraite à l'éther.

On obtient 52,5 g de base.

$[\alpha]_D^{20} = -57°5$ (solution 5% diméthylformamide)

51 g de base (0,16 mole) sont dissous dans 150 ml d'acétate d'éthyle. On ajoute une solution de 5,9 g d'acide chlorhydrique sec dans 65 ml d'acétate d'éthyle. Le chlorhydrate cristallise. Il est essoré, lavé à l'acétate d'éthyle et séché en étuve à 40°C. On obtient 41,5 g de produit fondant à 117—119°C.

$[\alpha]_D^{20} = -20°4$ (solution aqueuse 5%)

Exemple X

1-éthyl-2-[2-éthoxy-3,5-dichlorophénoxyéthyl]pyrrolidine

— *2-éthoxy 3,5-dichlorophénol*

Dans un ballon muni d'un réfrigérant à reflux on introduit 152 g de 2-éthoxyphénol (1,15 moles), 136 g d'anhydride acétique et 10 gouttes d'acide sulfurique concentré. La réaction est très exothermique, la température monte vers 80°C. La réaction calmée on chauffe au bain marie pendant 15 minutes. On neutralise l'acide sulfurique par addition d'acétate de sodium.

On ajoute 345 ml d'acide acétique et par portions, en maintenant la température entre 20 et 25°C, 271 g de 1,3-dichloro 5,5-diméthyl hydantoïne (1,15 moles + 20% d'excès). On chauffe la suspension à 50—55°C pendant 97 heures. Très rapidement toute l'hydantoïne est dissoute.

On verse la solution dans 4 litres d'eau. Le dérivé chloré, liquide, est décanté puis désacétylé immédiatement par chauffage au reflux, en présence de lessive de soude diluée, jusqu'à dissolution totale. La solution est diluée à l'eau et le phénol est précipité par addition d'acide chlorhydrique concentré. Il est décanté. La phase aqueuse est extraite à l'éther. La phase éthérée est séchée sur sulfate de sodium. Après élimination de l'éther, le 2-éthoxy 3,5-dichlorophénol est distillé sous vide. On recueille 166 g de produit qui distille à 130—4°C sous 15 mmHg (1995 Pa). Le produit cristallise. P.F. = 45°C.

— *1-éthyl-2-[2-éthoxy-3,5-dichlorophénoxyéthyl]pyrrolidine*

On prépare une solution d'éthylate de sodium à partir de 9,2 g de sodium et 120 ml d'éthanol absolu puis ajoute 83 g de 2-éthoxy 3,5-dichlorophénol (0,4 mole). On distille l'alcool puis ajoute 240 ml de xylène sec. On élimine les dernières traces d'alcool par distillation azéotropique. Après refroidissement on ajoute 71 g de 1-éthyl-2-[$\beta$-chloroéthyl]pyrrolidine (0,4 mole + 10% d'excès) et abandonne le mélange réactionnel pendant une nuit.

On chauffe au reflux 4 heures puis on refroidit et reprend le mélange par 600 ml d'eau et 30 ml d'acide chlorhydrique concentré. On décante la phase aqueuse, la filtre avec du noir puis précipite la base par addition de 60 ml d'ammoniaque concentré. Elle est décantée. La solution aqueuse est extraite au chlorure de méthylène. La phase organique est séchée sur carbonate de potassium. Après élimination du solvant le produit restant est distillé sous vide. On obtient 101 g de base.

P.E. = 198—200°C sous 8 mmHg (1064 Pa).

La base est dissoute dans 300 ml d'éthanol absolu. On ajoute une solution de 58,5 g d'acide citrique anhydre dans 200 ml d'éthanol. Le citrate formé est essoré, lavé à l'alcool et séché à l'air. P.F. = 95—100°C. Il est recristallisé avec passage au noir dans 130 ml d'éthanol. On obtient 124 g de citrate de 1-éthyl-2-[2-éthoxy-3,5-dichlorophénoxyéthyl]pyrrolidine. P.F. = 95—100°C.

Exemple XI

1-cyclohexyl 4-[2-méthoxy 3,5-dichlorophénoxy]hexaméthylène imine

1-cyclohexyl 2-[2-methoxy 3,5-dichlorophénoxyéthyl]pyrrolidine

Selon la technique de l'exemple I, on obtient par réaction de 116 g de 3,5-dichlorogaïacol (0,6 mole) et 148 g de 1-cyclohexyl-2-[2-chloroéthyl]pyrrolidine (0,6 mole + 11%) 233 g de produit (théorie 222 g) qui, d'après l'analyse C.C.M. est un mélange de 2 isomères:

— 1-cyclohexyl-4-[2-méthoxy 3,5-dichlorophénoxy]hexaméthylène imine

— 1-cyclohexyl-2-[2-méthoxy 3,5-dichlorophénoxyéthyl]pyrrolidine

On dissout 233 g de base dans 450 cc d'eau et 53 cc d'acide chlorhydrique concentré. On refroidit en amorçant la cristallisation et on abandonne le tout une nuit. On obtient 163 g de chlorhydrate. Ce chlorhydrate ne contient pratiquement que du dérivé hexaméthylène imino.

Ce chlorhydrate est recristallisé avec filtration sur noir, on obtient 150 g de chlorhydrate de 1-cyclohexyl-4-[2-méthoxy 3,5-dichlorophénoxy]hexaméthylène imine fondant à 174—176°C.

Les jus aqueux sont ensuite filtrés avec du noir et alcalinisés par addition d'ammoniaque à 20%. L'huile qui décante est extraite à l'éther. La solution éthérée obtenue est séchée sur carbonate de potassium puis l'éther est distillé en terminant sous vide jusqu'à poids constant.

Poids obtenu: 92 g

86 g de base (0,23 mole) sont dissous dans 260 ml d'acétonitrile et 53 g d'acide phosphorique (2 × 0,23 mole). Le phosphate qui se forme précipite sous forme d'une huile qui semble être cristallisée après une nuit de repos. Ce phosphate est essoré, lavé avec de l'acétonitrile, séché à l'air puis sous vide sur acide sulfurique mais le produit obtenu est à moitié cristallisé. Il est repris dans 150 ml d'éthanol absolu et abandonné une nuit. Il est alors bien cristallisé. Il est essoré, lavé à l'éthanol et séché à 40°C. On obtient 55 g de bis-phosphate de 1-cyclohexyl-2-[2-méthoxy 3,5-dichlorophénoxyéthyl]pyrrolidine fondant à 138—138,5°C.

Exemple XII

a) Chlorhydrate de 1-cyclopropylméthyl-2-(2-chloroéthyl) pyrrolidine

*1° — 1-cyclopropylméthyl-2-carbethoxyméthyl-pyrrolidone*

Dans un autoclave d'un litre, on introduit 78 g de cyclopropylméthylamine (1,1 mole) et 220 g de 3-hexène dioate d'éthyle (1,1 mole). On chauffe doucement à 165°C et maintient cette température pendant 8 heures. Après refroidissement, le produit est distillé sous vide. On élimine d'abord l'alcool formé puis recueille 155 g de produit distillant à 157—160° sous 3 mmHg (399 Pa).

*2° — 1-cyclopropylméthyl-2-(2-hydroxyéthyl)pyrrolidine*

Dans un ballon tricol de 3 litres muni d'un agitateur étanche d'un réfrigérant à reflux et d'une ampoule à brome, un introduit 52,5 g d'hydrure de lithium et d'aluminium (2 × 0,69 mole) et 390 ml de tétrahydrofuranne sec. A la suspension obtenue, on ajoute goutte à goutte et de manière à maintenir un léger reflux une solution de 155 g de 1-cyclopropylméthyl-2-carbethoxyméthyl-5-pyrrolidone (0,69 mole). L'introduction dure environ deux heures. On maintient ensuite le reflux pendant trois heures trente. Après refroidissement, on détruit l'excès d'hydrure de lithium et d'aluminium par addition lente (1 heure environ) de 75 ml d'eau. La réaction très exothermique nécessite un refroidissement extérieur. On ajoute à la suspension 1050 ml d'acide chlorhydrique 6 N puis 580 g de sel de Seignette et 580 ml d'eau.

On alcalinise avec 470 ml de lessive de soude à 30%. La phase organique est décantée, séchée sur $K_2CO_3$.

On évapore le tétrahydrofuranne puis distille sous vide le 1-cyclopropylméthyl-2-(2-hydroxy-éthyl)pyrrolidine.

On recueille 102 g (87,5%) de produit qui passe à 125—127°C sous 10 mmHg.

*3° — Chlorhydrate de 1-cyclopropylméthyl-2-(2-chloroéthyl)pyrrolidine*

Dans un ballon de trois litres muni d'un agitateur, d'un réfrigérant à reflux, d'un thermomètre et d'une ampoule à brome, un introduit 172 g de 1-cyclopropylméthyl-2-(2-hydroxyéthyl)pyrrolidine (1,02 mole) et 510 ml de chloroforme. On refroidit la solution à 10°C puis ajoute goutte à goutte en maintenant la température entre 10° et 15°C, 151,5 g de chlorure de thionyle (1,02 mole + 25%). On chauffe ensuite à reflux pendant 7 heures.

On distille le chloroforme sous vide jusqu'à poids constant. On obtient 254 g de chlorhydrate sous forme liquide.

La base est libérée au moment de l'emploi par dissolution du chlorhydrate dans l'eau, alcalinisation à la soude et extraction à l'éther.

On recueille ainsi 175 g d'amine (92%).

b) 1-cyclopropylméthyl-4-[2-méthoxy 3,5-dichlorophénoxy]hexaméthylène imine

1-cyclopropylméthyl-2-[2-méthoxy 3,5-dichlorophénoxyéthyl]pyrrolidine

En suivant la technique de l'Exemple I sus-mentionné, on obtient par réaction de 174 g de 3,5-dichloro gaïacol (0,90 mole) et 186 g de 1-cyclopropylméthyl-2-[2-chloroéthyl]pyrrolidine (0,90 mole + 10%) 329 g de produit (théorie 310 g) qui est d'après l'analyse C.C.M. un mélange de deux isomères:

1 cyclopropylméthyl-4-[2-méthoxy 3,5-dichlorophénoxy]hexaméthylène imine et cyclopropylméthyl-2-[2-méthoxy 3,5-dichlorophénoxyéthyl]pyrrolidine.

On dissout 328,5 g de base dans 500 ml d'éthanol et ajoute à la solution obtenue 173 g d'acide citrique (0,9 mole) dissous dans 1000 ml d'éthanol. Le citrate cristallise lentement. Il est essoré, lavé avec 300 ml d'éthanol et séché à 40°C. On obtient 389 g de citrate fondant à 60—65°C qui d'après l'analyse C.C.M. est un mélange plus riche en dérivé hexaméthylèneimino qu'en dérivé pyrrolidino.

Ce citrate est recristallisé avec filtration sur noir successivement dans 780 ml d'acétonitrile, 510 ml puis 645 ml de méthyléthylcétone. On obtient 189 g de 1-cyclopropylméthyl-4-[2-méthoxy 3,5-dichlorophénoxy]hexaméthylène imine fondant à 71—73°C. et ne présentant plus qu'une tache en C.C.M. Les jus alcooliques de cristallisation du citrate sont concentrés jusqu'à un volume de 280 ml. On dilue avec un litre d'eau et alcalinise la solution par addition d'ammoniaque à 20%. L'huile qui décante est extraite au chlorure de méthylène. La phase organique est séchée sur carbonate de potassium puis

le chlorure de méthylène est distillé en terminant sous vide jusqu'à poids constant. Poids obtenu = 76 g.

69 g de base (0,2 mole) sont dissout dans 275 ml d'éthanol absolu. On ajoute 23 g d'acide fumarique (0,2 mole) et chauffe jusqu'à dissolution.

On refroidit. Le fumarate qui cristallise est essoré, lavé avec 60 ml d'éthanol et séché en étuve. Poids obtenu = 65 g.

Ce produit est un mélange contenant en majorité du dérivé pyrrolidino.

Il est recristallisé avec filtration avec du noir dans 130 ml d'éthanol à 95°.

On obtient 45 g de fumarate de 1-cyclopropylméthyl-2-[2-méthoxy 3,5-dichlorophénoxyéthyl]-pyrrolidine fondant à 162—163°C. L'analyse C.C.M. ne permet pas de déceler de dérivé hexaméthylène imino.

## Exemple XIII

1-(cyclohexenylméthyl)-2-[2-(2-méthoxy-3,5-dichlorophenoxy)ethyl]pyrrolidine

1-(1-cyclohexenylmethyl)-4-(2-méthoxy-3,5-dichlorophenoxy)azepine

*1° — 2-[1-(1-cyclohexenylméthyl)-2-pyrrolidyl]ethanol*

Dans un ballon de 500 ml muni d'un agitateur, d'un thermomètre, d'un réfrigérant et d'une ampoule à brome on introduit: 16,4 g de 2-(2-pyrrolidyl) éthanol (0,143 mole) 84,3 ml de potasse alcoolique 1,78 N (0,150 mole) et goutte à goutte 28 g de 1-bromométhyl cyclohexène (pureté ~ 90%) (0,160 mole).

La température passe de 20° à 55°C et il se forme un précipité. On laisse réagir une heure puis filtre les sels et évapore à sec le filtrat.

L'huile résiduelle est reprise dans 150 ml d'eau. On acidifie à l'acide chlorhydrique jusqu'à pH = 1 et extrait avec deux fois 100 ml d'éther éthylique.

La phase aqueuse est alcalinisée à la soude puis extraite avec trois fois 100 ml d'éther. Ces extraits sont séchés sur sulfate de magnésie, filtrés puis évaporés à sec.

L'huile résiduelle est distillée sous vide: P.E. 2 mmHg (266 Pa): 100—107°C $n_D^{20}$ = 1,506 — Rendement: 20,7 g.

*2° — Chlorhydrate de 2-[1-(1-cyclohexenylméthyl)-2-pyrrolidinyl]chloro éthane.*

Dans un ballon de 250 ml muni d'un agitateur, d'un themomètre, d'un réfrigérant et d'une ampoule à brome, on introduit 17,8 g de 2-[1-(1-cyclohexenyl méthyl)-2-pyrrolidyl]éthanol (0,085 mole), 50 ml de chloroforme et goutte à goutte en maintenant la température vers 20—25°C par refroidissement 15,3 ml (25 g) de chlorure de thionyle. On chauffe ensuite à reflux pendant trois heures.

On évapore la solution à sec puis reprend le résidu dans 20 ml de toluène et évapore à nouveau à sec sous vide.

Le solide résiduel est mis en suspension dans 100 ml d'acétate d'éthyle.

Les cristaux sont filtrés et séchés en étuve à 50°C.

On obtient 19,4 g (87%) de produit fondant à 122°C.

*3° — 1-(1-cyclohexenylméthyl-2-[2-(2-méthoxy-3,5-dichlorophenoxy)éthyl]pyrrolidine*
*1-(1-cyclohexenylméthyl)-4-(2-méthoxy-3,5-dichlorophenoxy)azepine*

Dans un ballon de 250 ml muni d'un agitateur, d'un thermomètre, d'un réfrigérant et d'une ampoule à brome, on introduit 60 ml d'éthanol et par portions 1,4 g de sodium (0,060 atg). Après dissolution totale on ajoute 11,6 g de 3,5-dichloro gaïacol (0,060 mole) puis évapore le solvant sous vide.

Le résidu solide est dissous dans 60 ml de DMF, on chauffe la solution vers 100°C puis coule goutte à goutte une solution de 8 g de chlorhydrate de 2-[1-(1-cyclohexenylméthyl)-2-pyrrolidyl]-chloroéthane (0.030 mole) dans 40 ml de DMF.

On maintient le chauffage pendant deux heures puis évapore le solvant à sec sous vide.

Le résidu est repris dans 300 ml d'eau et le mélange est alcalinisé à l'ammoniaque. On extrait la suspension avec trois fois 100 ml d'éther puis lave la phase organique avec 100 ml d'eau. On sèche la solution éthérée sur sulfate de magnésie puis évapore le solvant à sec sous vide.

Poids: 12 g.

Le spectre RMN correspond à celui d'un mélange contenant environ 30% de composé pyrrolidinique et 70% de son isomère azépinique.

Le mélange est dissous dans 40 ml de méthyl éthyl cétone. On acidifie à pH = 1 avec une solution concentrée d'acide chlorhydrique dans l'isopropanol puis amorce la cristallisation et laisse reposer une nuit.

Les cristaux sont filtrés, lavés à l'isopropanol et séchés en étuve à 60°C. On obtient 7,3 g de produit fondant à ≃ 172°C. Le spectre RMN correspond à celui d'un mélange contenant 80 à 85% de composé azépinique. Le filtrat est évaporé à sec et le résidue est repris dans 65 ml d'isopropanol. Les cristaux blancs insolubles sont filtrés, lavés à l'isopropanol puis séchés en étuve à 50°C. On recueille 1 g de produit fondant à ≃ 160°C et dont le spectre RMN correspond au chlorhydrate de 1-(1-cyclohexenylméthyl)-2-[2-(2-méthoxy-3,5-dichlorophenoxy) éthyl] pyrrolidine.

Les produits selon l'invention sont utilisés sous forme de solution injectable, de collyre, de lotion dont la préparation est connue en soi.

Les composés de l'invention peuvent être utilisés en injection locale sous forme d'ampoules dosées à 10 mg/5 ml. On peut également appliquer les composés de l'invention localement sous forme de badigeonnages.

Les exemples suivants concernent des préparations pharmaceutiques réalisées de façon conventionnelle à partir des composés de l'invention.

Exemple A

Soluté injectable

| | | |
|---|---|---|
| Chlorhydrate de 1-éthyl-2-[2-méthoxy 3,5-dichloro-phénoxyéthyl]pyrrolidine | | 10 mg |
| Chlorure de potassium | | 44 mg |
| Eau pour préparations injectables | q.s. | 2 ml |

Exemple B

Collyre

| | | |
|---|---|---|
| Citrate de 1-méthyl-2-[2-méthoxy 3,5-dichloro-phenoxyéthyl]pyrrolidine | | 1,60 g |
| Parahydroxybenzoate de méthyle | | 1,30 g |
| Parahydroxybenzoate de propyle | | 0,20 g |
| Eau pour préparations injectables | q.s. | 1000 ml |

Exemple C

Lotion

| | | |
|---|---|---|
| Chlorhydrate de 1-éthyl-2-[2-méthoxy 3,5-dichloro-phénoxyéthyl]pyrrolidine | | 0,334 g |
| Alcool éthylique à 95° | | 52 g |
| Eau pour préparations injectables | q.s. | 100 ml |

Les composés de l'invention exercent d'intéressantes propriétés pharmacologiques sur le système nerveux central notamment comme anesthésiques locaux.

La toxicité des composés de l'invention a été déterminée chez la souris par voie parentérale (intraveineuse, intrapéritonéale, sous-cutanée) et par voie orale. Les léthales 50 sont rassemblées à titre d'exemple dans le tableau I.

Les composés de l'invention sont, dans le tableau I et dans les suivants, numérotés comme ci-après.

| COMPOSES | NUMEROS |
|---|---|
| Fumarate de 1-méthyl-2-[2-méthoxy 3,5-dichloro-phénoxyéthyl]pyrrolidine (racémique) | Composé 1 |
| 1-méthyl-2-[2-méthoxy 3,5-dichlorophénoxyéthyl] pyrrolidine (dextrogyre) | Composé 2 |
| 1-méthyl-2-[2-méthoxy 3,5-dichloro-phénoxyéthyl]pyrrolidine (lévogyre) | Composé 3 |
| Chlorhydrate de 1-éthyl-2-[2-méthoxy 3,5-dichloro-phénoxyéthyl]pyrrolidine (racémique) | Composé 4 |
| Chlorhydrate de 1-éthyl-2-[2-méthoxy 3,5-dichloro-phénoxyéthyl]pyrrolidine (dextrogyre) | Composé 5 |
| Chlorhydrate de 1-éthyl-2-[2-méthoxy 3,5-dichloro-phénoxyéthyl]pyrrolidine (lévogyre) | Composé 6 |
| Fumarate de 1-allyl-2-[2-méthoxy 3,5-dichloro-phénoxyéthyl]pyrrolidine (racémique) | Composé 7 |
| Citrate de 1-éthyl-2-[2-éthoxy-3,5-dichloro-phénoxyéthyl]pyrrolidine (racémique) | Composé 8 |
| 1-éthyl-2-[3,5-dichlorophénoxy-éthyl]pyrrolidine (racémique) | Composé 9 |
| Fumarate de 1-méthyl-2-[2-méthoxy 3,5-dibromo-phénoxyéthyl]pyrrolidine (racémique) | Composé 10 |
| Chlorhydrate de 1-méthyl-4-[2-méthoxy 3,5-dichloro-phénoxy]hexaméthylène imine | Composé 11 |
| Citrate de 1-allyl-4-[2-méthoxy 3,5-dichloro-phénoxy]hexaméthylène imine | Composé 12 |
| Chlorhydrate de 1-cyclohexyl-4-[2-méthoxy 3,5-dichloro-phénoxy]hexaméthylène imine | Composé 13 |
| Bis-phosphate de 1-cyclohexyl 2-[2-méthoxy 3,5-dichloro-phénoxyéthyl]pyrrolidine | Composé 14 |
| Citrate de 1-cyclopropylméthyl 4-[2-méthoxy-3,5-dichlorophénoxy]hexaméthylène imine | Composé 15 |
| Fumarate de 1-cyclopropylméthyl 2-[2-méthoxy-3,5-dichlorophénoxyéthyl]pyrrolidine | Composé 16 |

TABLEAU I — TOXICITE

DL 50 — SOURIS — mg/Kg (base)

| COMPOSES | IV | IP | C | PO |
|---|---|---|---|---|
| 1 | 34,7—36,5 | 160—192 | 597—651 | 521—561 |
| 2 | 27,5—28,2 | 161—168 | 702—800 | 543—561 |
| 3 | 44,9—45,2 | 182—192 | 543 | 434—486 |
| 4 | 35—32,3 | 137—141 | 365—377 | 574—597 |
| 5 | 29,6—30,9 | 149—156 | 464—487 | 565—574 |
| 6 | 32,7—35,5 | 151—157 | 377—381 | 484—518 |
| 7 | 28,9—32,2 | 186—189 | 777—821 | 479—518 |
| 8 | 25,3—25,7 | 152—160 | 469—488 | 684—689 |
| 9 | 36,4—37,3 | 117—119 | 248—257 | 244—253 |
| 10 | 43,2—45,7 | 149—154 | 382—431 | 340—371 |
| 11 | 34,3—36,3 | 168—169 | 248—271 | 643—679 |
| 12 | 31,6—36 | 182—195 | 60% mortalité à 1400 mg/Kg | 480—531 |
| 13 | 15,3—14,8 | 91,1—86,5 | 10% mortalité à 180 mg/Kg | 455—464 |
| 14 | 19,7—19,3 | 88,4—88 | 550—591 | 206—216 |
| 15 | 18,5—19,1 | 122—116 | 411—404 | 270—254 |
| 16 | 22,9—26,9 | 142—136 | 479—512 | 293—303 |

Les propriétés anesthésiques locales des composés de l'invention ont été mises en évidence d'après les différents tests décrits ci-après.

L'anesthésie locale de surface a été déterminée par la méthode de Règnier décrite dans la "thèse Doc Med Paris 1929".

Cette méthode consiste à étudier la suppression du réflex oculopalpébral sur la cornée du lapin.

Sur un lot de 10 lapins on mesure la profondeur de l'anesthésie cornéenne obtenue après instillation dans l'oeil de 2 gouttes de la solution aqueuse du produit à étudier par comparaison à celle produite par des solutions aqueuses de chlorhydrate de cocaïne à des concentrations différentes.

L'expérience est réalisée en essai croisé. Le nombre moyen horaire de coups de crin sur la cornée ne produisant aucune réaction indique l'intensité de l'anesthésie. On peut donc évaluer le pourcentage d'anesthésie en fonction de la concentration et déterminer graphiquement la CE 50.

On appelle CE 50 la concentration d'une solution qui injectée, sous un volume donné abolit la réponse sensitive chez 50% des animaux.

Les index d'activité qui sont définis par

$$\text{LE RAPPORT} = \frac{\text{CE 50 de l'anesthésique de référence}}{\text{CE 50 de produit à étudier}}$$

sont mentionnés dans le tableau II.

TABLEAU II

INDEX D'ACTIVITE DE L'ANESTHESIE DE SURFACE CHEZ LE LAPIN

| COMPOSES | 1 | 2 | 3 | 4 | 5 | 6 | 7 | 11 | 16 |
|---|---|---|---|---|---|---|---|---|---|
| Index d'activité | 1,36 | 1,72 | 1,92 | 4,65 | 3,52 | 2,12 | 1,84 | 1,56 | 5,68 |

L'activité anesthésique de conduction a été réalisée de la façon suivante: un anesthésique local injecté en profondeur dans la cuisse du rat le long du trajet du sciatique, à raison de 1 ml provoque une anesthésie des terminaisons nerveuses que l'on caractérise et mesure en pinçant les orteils médians de la patte postérieure du rat.

Les composés de l'invention ou l'anesthésique de référence (xylocaïne) sont injectés sous un volume de 1 ml pour une concentration donnée à un lot de dix rats mâles.

30 minutes, 1 heure, 2 heures, 3 heures après l'administration on pince les 3 orteils médians de la patte postérieure et on note les réponses positives de l'animal à chaque pincement d'orteil.

La sommation des réponses positives des dix animaux permet d'obtenir le pourcentage d'anesthésie. La CE 50 est ensuite déterminée graphiquement.

Les index d'activité sont donnés dans le tableau III.

TABLEAU III

INDEX D'ACTIVITE DE L'ANESTHESIE DE CONDUCTION CHEZ LE RAT

| COMPOSES | 1 | 2 | 3 | 4 | 5 | 6 | 8 | 11 | 12 |
|---|---|---|---|---|---|---|---|---|---|
| Index d'activités | 3,31 | 4 | 2,1 | 2,4 | 4,14 | 3,4 | 2,44 | 2,4 | 2,8 |

L'anesthésie d'infiltration a été étudiée chez le cobaye selon la méthode décrite par Bulbring et Wajda dans J. Pharmacol. Exp. Ther. (1945) *85* 78—84.

Cette méthode repose sur la disparition du réflex peaucier provoqué chez le cobaye par un stimulus mécanique.

Nous avons opéré sur des lots de 10 cobayes adultes mâles dont on a, la veille épilé soigneusement le dos sur une surface de 16 $cm^2$. On délimite à l'encre 4 zones ABCD telles que: A = zone antérieure gauche; B = zone antérieure droite; C = zone postérieure gauche; D = zone postérieure droite.

Au centre de chaque zone, on vérifie l'apparition du réflex peaucier en réponse à une seule excitation provoquée par une épingle. On injecte ensuite 0,2 ml de la solution d'anesthésique dans le soluté isotonique injectable de chlorure de sodium. Cinq minutes après on excite régulièrement le centre du bouton intradermique obtenu au rythme d'une excitation toutes les 3 secondes jusqu'à l'apparition du réflex ou si l'anesthésie est totale un numbre de fois égal à 6. On continue l'exploration de l'anesthésie toutes les 5 minutes pendant 30 minutes.

Le produit de référence est la procaïne.

Sur un lot de 10 cobayes, pour une concentration donnée de procaïne et de composé de l'invention, on teste 5 animaux en A et C avec le composé de l'invention et le reste B et D avec la procaïne; le lendemain on pratique un essai croisé en inversant les zones d'injection.

Sur deux autres lots on répète la même expérimentation avec deux autres concentrations, soit du composé de l'invention, soit de la procaïne, on calcule pour chaque concentration la moyenne des excitations obtenues sur 10 cobayes.

Le pourcentage d'anesthésie en fonction de la concentration nous permet de déterminer graphiquement la CE 50.

Les index d'activité sont donnés dans le tableau IV.

TABLEAU IV

INDEX D'ACTIVITE DE L'ANESTHESIE D'INFILTRATION CHEZ LE COBAYE

| COMPOSES | 1 | 2 | 3 | 5 | 6 | 8 | 9 | 10 | 14 | 16 |
|---|---|---|---|---|---|---|---|---|---|---|
| Index d'activité | 3,58 | 3,1 | 4,2 | 1,65 | 2,69 | 5 | 1,8 | 3,17 | 4,07 | 2,55 |

Les résultats de ces tests prouvent une activité anesthésique des composés de l'invention 2 à 4

fois supérieure en moyenne à celle des anesthésiques de référence (xylocaïne, procaïne, cocaïne).

L'intérêt suscité par les expérimentations pratiquées chez les animaux de laboratoire s'est vu très largement justifié lors des essais en clinique humaine des composés de l'invention. A titre d'exemple on peut citer:

— Le cas d'une malade de 68 ans atteinte de maladie post-phlébitique avec importants troubles trophiques bilatéraux sus-malléolaires internes.

Ces troubles ont provoqué des ulcéres de jambe anciens, actuellement cicatrisés, mais siège d'un prurit intense à recrudescence nocturne avec lichenification. L'application locale de lotion de 1-éthyl-2-[2-méthoxy-3,5-dichlorophénoxyéthyl]pyrrolidine à 0,3%, provoque une disparition du prurit de plus en plus importante et surtout de plus en plus prolongée après chaque application, à tel point que les lésions de grattage disparaissent, ce qui provoque la disparition du lichen.

— Une patiente de 38 ans a dû subir une oestéosynthèse du tibia gauche à la suite d'un accident de ski. La guérison orthopédique est parfaite et la récupération excellente. Cependant, la malade est très gênée par un "prurit ferox", sine materia au niveau de la cicatrice.

L'application de lotion de 1-méthyl-2-[2-méthoxy-3,5-dichlorophénoxyéthyl]pyrrolidine à 0,3% provoque des le ler jour une atténuation considérable du prurit qui disparaît totalement en une semaine.

— Un sujet de 67 ans souffre depuis plusieurs mois d'un prurit anal atroce, entraînant: anorexie, insomnie, faisant évoquer le suicide.

Tous les traitements proctologiques et dermatologiques sont restés inefficaces.

En 3 jours, l'application continue de compresses imbibées de lotion de 1-éthyl-2-[2-éthoxy-3,5-dichlorophénoxyéthyl]pyrrolidine à 0,3% fait disparaître totalement et définitivement le prurit: cette amélioration provoque une reprise considérable de l'appétit à tel point que le malade acquiert un embonpoint inconnu jusqu'à ce jour.

— Un jeune homme de 17 ans présente une maladie de Hodgkin stade IV, dont un des signes révélateurs a été un prurit intense prédominant sur la face antérieure des deux avant-bras.

Parallèlement au traitement chimiothérapique, on lui conseille des badigeons au pinceau avec la solution de 1-méthyl-2-[2-méthoxy 3,5-dibromophénoxyéthyl]pyrrolidine.

Le soulagement, bien qu'incomplet est satisfaisant et permet le retour du sommeil.

— Chez une patiente de 47 ans l'instillation de deux gouttes de solution 1-éthyl-2-[3,5-dichlorophénoxyéthyl]pyrrolidine permet l'extraction facile d'un corps étranger métallique incrusté dans la conjonctive.

**Revendications pour les Etats contractants: BE CH DE FR GB IT LI LU NL SE**

1. Nouvelles phénoxyamines hétérocycliques substituées de formule générale I:

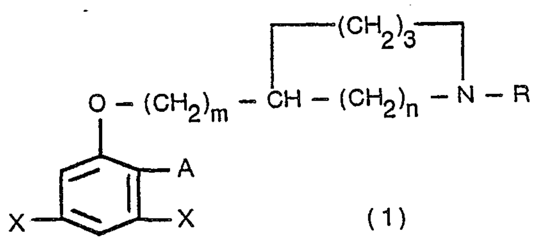

(1)

Dans laquelle m = 0 ou 2
n = 0 ou 2
à la condition que m + n = 2
et dans laquelle:

A = — hydrogène,

— alkoxy comportant 1 à 4 atomes de carbone, tel que méthoxy ou éthoxy, propoxy ou butoxy linéaire ou ramifié,

— alcényloxy comportant 2 à 6 atomes de carbone, tel que vinyloxy, propènyloxy (allyloxy), butényloxy, pentènyloxy, hexènyloxy,

X = — halogène tel que F, Cl, Br,

R = — hydrogène,

— alkyle inférieur comportant 1 à 6 atomes de carbone tel que méthyle ou éthyle, propyle, butyle, pentyle ou hexyle linéaire ou ramifié,

— cycloalkyle comportant 3 à 6 atomes de carbone et pouvant être substitué par un groupe alkyle comportant 1 ou 2 atomes de carbone, tel que cyclopropyle, cyclobutyle, cyclopentyle, cyclohexyle, méthylcyclobutyle, méthylcyclopentyle, méthylcyclohexyle, éthylcyclohexyle,

— alcènyle comportant 3 à 6 atomes de carbone, tel que propèn-2-yle (allyle), butènyle, pentènyle, hexènyle,

— cycloalcènyle comportant 4 à 6 atomes de carbone et pouvant être substitué par un groupe

alkyle comportant 1 ou 2 atomes de carbone, tel que cyclobutènyle, cyclopentènyle, cyclohexènyle, méthyl cyclobutènyle, méthylcyclopentènyle, méthylcyclohexènyle, éthylcyclohexènyle,

— cycloalkyle- ou cycloalcènyle-alkyle où les groupes cycloalkyle, cycloalcènyle et alkyle sont définis comme précédemment, tel que cyclopropylméthyle, cyclopropyléthyle, cyclobutylméthyle, cyclobutyléthyle, cyclopentylméthyle, cyclopentyléthyle, cyclopentènylméthyle, cyclopentènyléthyle, cyclohexylméthyle, cyclohexyléthyle, cyclohexènylméthyle, cyclohexènyléthyle.

2. Nouveaux composés selon la revendication 1 caractérisés en ce qu'ils sont sous forme de sels d'addition avec un acide minéral ou organique, de sels d'ammonium quaternaire ou de N-oxydes.

3. Un composé selon l'une des revendications 1 ou 2 caractérisé en ce qu'il est sous forme d'un isomère optiquement actif.

4. Composé selon l'une des revendications 1, 2 ou 3, caractérisé par la formule générale:

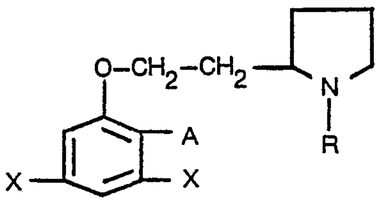

dans laquelle R, A et X ont la même définition que dans ces revendications.

5. Composé selon l'une des revendications 1, 2 ou 3, caractérisé par la formule générale:

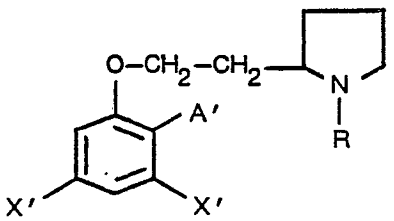

dans laquelle:

A' représente H, $OCH_3$, $OC_2H_5$

X' représente Cl ou Br

R est comme défini comme précédemment.

6. Suivant l'une des revendications 1 à 5, le chlorhydrate de 1-éthyl-2-[2-méthoxy 3,5-dichlorophénoxyéthyl]pyrrolidine.

7. Suivant l'une des revendications 1 à 5, le chlorhydrate de 1-éthyl-2-[2-méthoxy 3,5-dichlorophénoxyéthyl]pyrrolidine dextrogyre.

8. Suivant l'une des revendications 1 à 5, le chlorhydrate de 1-éthyl-2-[2-méthoxy 3,5-dichlorophénoxyéthyl]pyrrolidine lévogyre.

9. Suivant l'une des revendications 1 à 5, le fumarate de 1-méthyl-2-[2-méthoxy 3,5-dichlorophénoxyéthyl]pyrrolidine.

10. Suivant l'une des revendications 1 à 5, la 1-méthyl-2-[2-méthoxy 3,5-dichlorophénoxyéthyl]-pyrrolidine dextrogyre.

11. Suivant l'une des revendications 1 à 5, la 1-méthyl-2-[2-méthoxy 3,5-dichlorophénoxyéthyl]-pyrrolidine lévogyre.

12. Suivant l'une des revendications 1 à 5, le fumarate de 1-allyl-2-[2-méthoxy 3,5-dichlorophénoxyéthyl]pyrrolidine.

13. Suivant l'une des revendications 1 à 5, la 1-éthyl-2-[3,5-dichlorophénoxyéthyl]pyrrolidine.

14. Suivant l'une des revendications 1 à 5, le fumarate de 1-méthyl-2-[2-méthoxy 3,5-dibromophénoxyéthyl]pyrrolidine.

15. Suivant l'une des revendications 1 à 5, le citrate de 1-éthyl-2-[2-éthoxy 3,5-dichlorophénoxyéthyl]pyrrolidine.

16. Suivant l'une des revendications 1 à 5, le bis-phosphate de 1-cyclohexyl-2-[2-méthoxy-3,5-dichlorophénoxyéthyl]pyrrolidine.

17. Suivant l'une des revendications 1 à 5, le fumarate de 1-cyclopropyl-méthyl-2-[2-méthoxy 3,5-dichlorophénoxyéthyl]pyrrolidine.

18. Suivant l'une des revendications 1 à 5, le chlorhydrate de 1-(1-cyclohexénylméthyl)-2-[2-(2-méthoxy-3,5-dichlorophénoxy)éthyl]pyrrolidine.

19. Suivant l'une des revendications 6, 7, 8, 9, 12, 14, 15, 16, 17, 18, la phénoxyamine libérée de son sel.

20. A titre de médicaments nouveaux, à activité anesthésique locale, les composés selon les revendications 1 à 19.

21. Compositions pharmaceutiques caractérisées en ce qu'elles contiennent comme principe actif, l'un des composés selon les revendications 1 à 19 joint à des excipients pharmaceutiquement acceptables.

22. Procédé de préparation des composés suivant les revendications 1 à 18 qui consiste à traiter un phénol 3,5-dihalogéné de formule V:

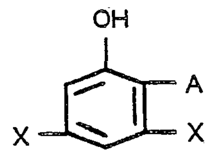

(V)

dans laquelle A et X sont définis comme dans la revendication 1, sous forme de phénolate alcalin avec une amine de formule VI:

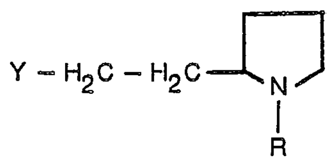

(VI)

dans laquelle Y est un reste anionique susceptible d'être éliminé et R est défini comme dans la revendication 1.

23. Procédé d'obtention des formes optiquement actives des composés de formule I par combinaison chimique avec un acide optiquement actif.

**Revendications pour l'Etat contractant: AT:**

1. Procédé pour la préparation de phenoxyamines hétérocycliques substituées de formule générale (I):

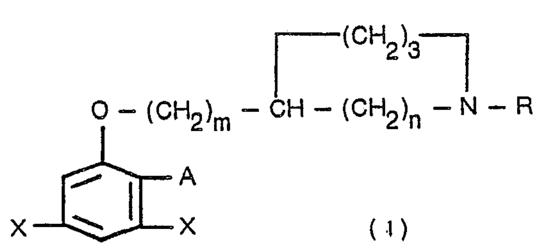

(I)

Dans laquelle m = 0 ou 2
n = 0 ou 2
à la condition que m + n = 2
et dans laquelle:
A = — hydrogène,
— alkoxy comportant 1 à 4 atomes de carbone, tel que méthoxy ou éthoxy, propoxy ou butoxy linéaire ou ramifié,
— alcényloxy comportant 2 à 6 atomes de carbone, tel que vinyloxy, propènyloxy (allyloxy), butényloxy, pentènyloxy, hexènyloxy,
X = — halogène tel que F, Cl, Br,
R = — hydrogène,
— alkyle inférieur comportant 1 à 6 atomes de carbone tel que méthyle ou éthyle, propyle, butyle, pentyle ou hexyle linéaire ou ramifié,
— cycloalkyle comportant 3 à 6 atomes de carbone et pouvant être substitué par un groupe alkyle comportant 1 ou 2 atomes de carbone, tel que cyclopropyle, cyclobutyle, cyclopentyle, cyclohexyle, méthylcyclobutyle, méthylcyclopentyle, méthylcyclohexyle, éthylcyclohexyle,
— alcènyle comportant 3 à 6 atomes de carbone, tel que propèn-2-yle (allyle), butènyle, pentènyle, hexènyle,
— cycloalcènyle comportant 4 à 6 atomes de carbone et pouvant être substitué par un groupe alkyle comportant 1 ou 2 atomes de carbone, tel quel cyclobutènyle, cyclopentènyle, cyclohexènyle, méthyl cyclobutènyle, méthylcyclopentènyle, méthylcyclohexènyle, éthylcyclohexènyle,
— cycloalkyle- ou cycloalcènyle-alkyle où les groupes cycloalkyle, cycloalcènyle et alkyle sont

définis comme précédemment, tel que cyclopropylméthyle, cyclopropyléthyle, cyclobutylméthyle, cyclobutyléthyle, cyclopentylméthyle, cyclopentyléthyle, cyclopentènylméthyle, cyclopentènyléthyle, cyclohexylméthyle, cyclohexyléthyle, cyclohexènylméthyle, cyclohexènyléthyle.
qui consiste à traiter un phénol 3,5-dihalogéné de formule:

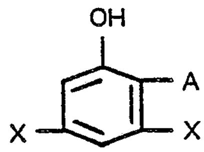

dans laquelle A et X sont définis comme dans la revendication 1, sous forme de phénolate alcalin avec une amine de formule:

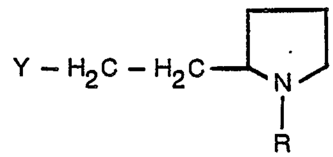

dans laquelle Y est un reste anionique susceptible d'être éliminé et R est défini comme dans la revendication 1.

2. Procédé de préparation selon la revendication, 1, caractérisé en ce que les phénoxyamines de formule (I) sont transformées en sels d'addition avec un acide minéral ou organique, en sels d'ammonium quaternaire avec un sel d'alkyle, ou oxydées en N-oxydes.

3. Procédé de préparation selon les revendications 1 ou 2 caractérisé en l'obtention des formes optiquement actives des composés de formule (I) par combinaison chimique avec un acide optiquement actif.

4. Procédé de préparation selon les revendications 1, 2 ou 3, des composés de formule générale (I) ayant la structure (II).

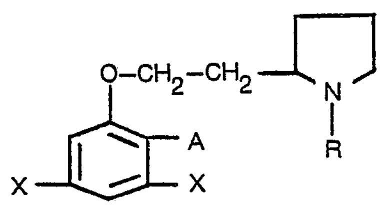

(II)

dans laquelle R, A et X ont la même définition que dans ces revendications.

5. Procédé de préparation selon l'une des revendications 1, 2 ou 3, des composés de formule générale (I) ayant la structure (III):

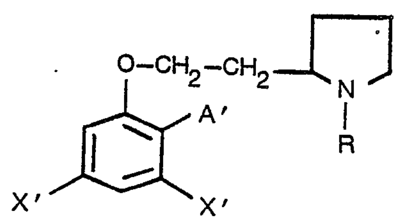

(III)

dans laquelle:

A' représente H, $OCH_3$, $OC_2H_5$

X' représente Cl ou Br

R étant comme défini comme précédemment.

6. Suivant l'une des revendications 1, 2, 4 ou 5, procédé de préparation du chlorhydrate de 1-éthyl-2[2-méthoxy 3,5-dichlorophénoxyéthyl]pyrrolidine.

7. Suivant l'une des revendications 1 à 5 procédé de préparation du chlorhydrate de 1-éthyl-2[2-méthoxy 3,5-dichlorophénoxyéthyl]pyrrolidine dextrogyre.

8. Suivant l'une des revendications 1 à 5 procédé de préparation du chlorhydrate de 1-éthyl-2[2-méthoxy 3,5-dichlorophénoxyéthyl]pyrrolidine lévogyre.

9. Suivant l'une des revendications 1, 2, 4 ou 5, procédé de préparation du fumarate de 1-méthyl-2-[2-méthoxy 3,5-dichlorophénoxyéthyl]pyrrolidine.

10. Suivant l'une des revendications 1 à 5, procédé de préparation de la 1-méthyl-2-[2-méthoxy

3,5-dichlorophénoxyéthyl]pyrrolidine dextrogyre.

11. Suivant l'une des revendications 1 à 5, procédé de préparation de la 1-méthyl-2-[2-méthoxy 3,5-dichlorophénoxyéthyl]pyrrolidine lévogyre.

12. Suivant l'une des revendications 1, 2, 4 ou 5, procédé de préparation du fumarate de 1-allyl-2-[2-méthoxy 3,5-dichlorophénoxyéthyl]pyrrolidine.

13. Suivant l'une des revendications 1, 2, 4 ou 5, procédé de préparation de la 1-éthyl-2-[3,5-dichlorophénoxyéthyl pyrrolidine.

14. Suivant l'une des revendications 1, 2, 4 ou 5, procédé de préparation du fumarate de 1-méthyl-2-[2-méthoxy 3,5-dibromophénoxyéthyl]pyrrolidine.

15. Suivant l'une des revendications 1, 2, 4 ou 5, procédé de préparation du citrate de 1-éthyl-2-[2-éthoxy 3,5-dichlorophénoxyéthyl]pyrrolidine.

16. Suivant l'une des revendications 1, 2, 4 ou 5, procédé de préparation du bis-phosphate de 1-cyclohexyl-2-[2-méthoxy-3,5-dichlorophénoxyéthyl]pyrrolidine.

17. Suivant l'une des revendications 1, 2, 4 ou 5, procédé de préparation du fumarate de 1-cyclopropylméthyl-2-[2-méthoxy 3,5-dichlorophénoxyéthyl]pyrrolidine.

18. Suivant l'une des revendications 1, 2, 4 ou 5, procédé de préparation du chlorhydrate de 1-(1-cyclohexénylméthyl)-2-[2-(2-méthoxy-3,5-dichlorophénoxy)éthyl]pyrrolidine.

**Patentansprüche für die Vertragsstaaten: BE CH DE FR GB IT LI LU NL SE**

1. Neue substituierte heterocyclische Phenoxyamine der allgemeinen Formel I:

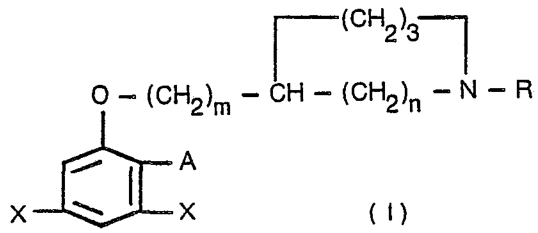

(I)

in der m = 0 oder 2 und
n = 0 oder 2 ist,
wobei die Bedingung m + n = 2 gilt, und in der
A = — Wasserstoff,
— Alkoxy mit 1—4 Kohlenstoffatomen, z.B. Methoxy oder Ethoxy, geradkettiges oder verzweigtes Propoxy oder Butoxy,
— Alkenyloxy mit 2 bis 6 Kohlenstoffatomen, z.B. Vinyloxy, Propenyloxy (Allyloxy), Butenyloxy, Pentenyloxy, Hexenyloxy,
X = — Halogen, z.b. F, Cl, Br,
R = — Wasserstoff,
— niedriges Alkyl mit 1 bis 6 Kohlenstoffatomen, z.B. Methyl oder Ethyl, geradkettiges oder verzweigtes Propyl, Butyl, Pentyl oder Hexyl,
— Cycloalkyl mit 3 bis 6 Kohlenstoffatomen, das mit einer 1 oder 2 Kohlenstoffatome enthaltenden Alkylgruppe substituiert sein kann, z.B. Cyclopropyl, Cyclobutyl, Cyclopentyl, Cyclohexyl, Methylcyclobutyl, Methylcyclopentyl, Methylcyclohexyl, Ethylcyclohexyl,
— Alkenyl mit 3 bis 6 Kohlenstoffatomen, z.B. Propen-2-yl (Allyl), Butenyl, Pentenyl, Hexenyl,
— Cycloalkenyl mit 4 bis 6 Kohlenstoffatomen, das mit einer 1 oder 2 Kohlenstoffatome aufweisenden Alkylgruppe substituiert sein kann, z.B. Cyclobutenyl, Cyclopentenyl, Cyclohexenyl, Methylcyclobutenyl, Methylcyclopentenyl, Methylcyclohexenyl, Ethylcyclohexenyl,
— Cycloalkyl- oder Cycloalkenyl-alkyl, wobei die Cycloalkyl-, Cycloalkenyl- und Alkylgruppen wie oben definiert sind, z.B. Cyclopropylmethyl, Cyclopropylethyl, Cyclobutylmethyl, Cyclobutylethyl, Cyclopentylmethyl, Cyclopentylethyl, Cyclopentenylmethyl, Cyclopentenylethyl, Cyclohexylmethyl, Cyclohexylethyl, Cyclohexenylmethyl, Cyclohexenylethyl, bedeuten.

2. Neue Verbindng nach Anspruch 1, dadurch gekennzeichnet, daß sie in Form ihrer Additionssalze mit einer Mineral- oder organischen, Säure, als quartäre Ammoniumsalze oder als N-Oxyde vorliegen.

3. Verbindung nach Anspruch 1 oder 2, dadurch gekennzeichnet, daß sie in Form eines optisch aktiven Isomeren vorliegt.

4. Verbindung nach einem der Ansprüche 1, 2 oder 3, gekennzeichnet durch die allgemeine Formel:

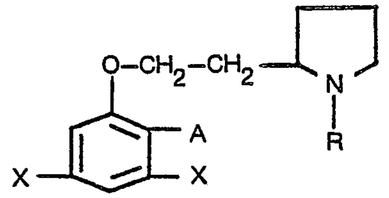

in der R, A und X wie oben definiert sind.

5. Verbindung nach einem der Ansprüche 1, 2 oder 3, gekennzeichnet durch die allgemeine Formel:

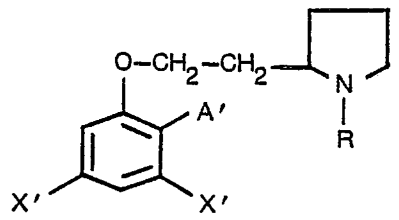

in der A' H, $OCH_3$ oder $OC_2H_5$ sowie
X' Cl oder Br bedeuten und
R wie oben definiert ist.

6. 1-Ethyl-2-[2-methoxy-3,5-dichlorophenoxyethyl]-pyrrolidin-hydrochlorid gemäß einem der Ansprüche 1 bis 5.

7. Rechtsdrehendes 1-Ethyl-2-[2-methoxy-3,5-dichlorophenoxyethyl]-pyrrolidin hydrochlorid gemäß einem der Ansprüche 1 bis 5.

8. Linksdrehendes 1-Ethyl-2-[2-methoxy-3,5-dichlorophenoxyethyl]-pyrrolidin hydrochlorid gemäß einem der Ansprüche 1 bis 5.

9. 1-Methyl-2-[2-methoxy-3,5-dichlorophenoxyethyl]-pyrrolidin-fumarat gemäß einem der Ansprüche 1 bis 5.

10. Rechtsdrehendes 1-Methyl-2-[2-methoxy-3,5-dichlorophenoxyethyl]-pyrrolidin gemäß einem der Ansprüche 1 bis 5.

11. Linksdrehendes 1-Methyl-2-[2-methoxy-3,5-dichlorophenoxyethyl]-pyrrolidin gemäß einem der Ansprüche 1 bis 5.

12. 1-Allyl-2-[2-methoxy-3,5-dichlorophenoxyethyl]-pyrrolidin-fumarat gemäß einem der Ansprüche 1 bis 5.

13. 1-Ethyl-2-[3,5-dichlorophenoxyethyl]-pyrrolidin gemäß einem der Ansprüche 1 bis 5.

14. 1-Methyl-2-[2-methoxy-3,5-dibromophenoxyethyl]-pyrrolidin-fumarat gemäß einem der Ansprüche 1 bis 5.

15. 1-Ethyl-2-[2-ethoxy-3,5-dichlorophenoxyethyl]-pyrrolidin-citrat gemäß einem der Ansprüche 1 bis 5.

16. 1-Cyclohexyl-2-[2-methoxy-3,5-dichlorophenoxyethyl]-pyrrolidin-diphosphat gemäß einem der Ansprüche 1 bis 5.

17. 1-Cyclopropylmethyl-2-[2-methoxy-3,5-dichlorophenoxyethyl]-pyrrolidin-fumarat gemäß einem der Ansprüche 1 bis 5.

18. 1-(1-Cyclohexenylmethyl)-2-[2-(2-methoxy-3,5-dichlorophenoxy)ethyl]-pyrrolidin-chlorhydrat gemäß einem der Ansprüche 1 bis 5.

19. Phenoxyamin gemäß einem der Anspruch 6, 7, 8, 9, 12, 14, 15, 16, 17 oder 18, freigesetzt aus seinem Salz.

20. Verbindungen gemäß einem der Ansprüche 1 bis 19 als neue Arzneimittel mit lokalanästhetischer Wirkung.

21. Pharmazeutische Zusammensetzungen, dadurch gekennzeichnet, daß sie als Wirkstoff eine der Verbindungen gemäß den Ansprüchen 1 bis 19 zusammen mit pharmazeutisch verträglichen Exzipienten enthalten.

22. Verfahren zur Herstellung der Verbindungen nach einem der Ansprüche 1 bis 18, dadurch gekennzeichnet, daß man ein 3,5-Dihalogenphenol der Formel V:

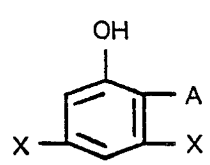

(V)

in der A und X wie in Anspruch 1 definiert sind, in Form eines Alkaliphenolats mit einem Amin der Formel VI:

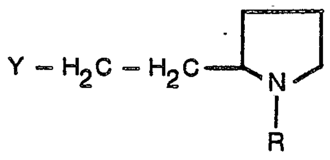

(VI)

in der Y ein anionischer, eliminierbarer Rest und R wie in Anspruch 1 definiert ist, umsetzt.

23. Verfahren zur Herstellung optisch aktiver Formen der Verbindungen der Formel I, gekennzeichnet durch eine chemische Umsetzung mit einer optisch aktiven Säure.

**Patentansprüche für den Vertragsstaat: AT**

1. Verfahren zur Herstellung von neuen substituierten heterocyclischen Phenoxyaminen der allgemeinen Formel I:

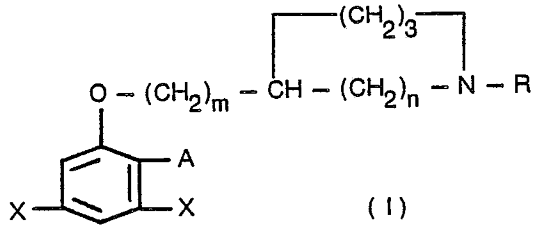

(I)

in der m = 0 oder 2 und
n = 0 oder 2 ist,
wobei die Bedingung m + n = 2 gilt, und in der
A = — Wasserstoff,
— Alkoxy mit 1—4 Kohlenstoffatomen, z.B. Methoxy oder Ethoxy, geradkettiges oder verzweigtes Propoxy oder Butoxy,
— Alkenyloxy mit 2 bis 6 Kohlenstoffatomen, z.B. Vinyloxy, Propenyloxy (Allyloxy), Butenyloxy, Pentenyloxy, Hexenyloxy,
X = — Halogen, z.b. F, Cl, Br,
R = — Wasserstoff,
— niedriges Alkyl mit 1 bis 6 Kohlenstoffatomen, z.B. Methyl oder Ethyl, geradkettiges oder verzweigtes Propyl, Butyl, Pentyl oder Hexyl,
— Cycloalkyl mit 3 bis 6 Kohlenstoffatomen, das mit einer 1 oder 2 Kohlenstoffatome enthaltenden Alkylgruppe substituiert sein kann, z.B. Cyclopropyl, Cyclobutyl, Cyclopentyl, Cyclohexyl, Methylcyclobutyl, Methylcyclopentyl, Methylcyclohexyl, Ethylcyclohexyl,
— Alkenyl mit 3 bis 6 Kohlenstoffatomen, z.B. Propen-2-yl (Allyl), Butenyl, Pentenyl, Hexenyl,
— Cycloalkenyl mit 4 bis 6 Kohlenstoffatomen, das mit einer 1 oder 2 Kohlenstoffatome aufweisenden Alkylgruppe substituiert sein kann, z.B. Cyclobutenyl, Cyclopentenyl, Cyclohexenyl, Methylcyclobutenyl, Methylcyclopentenyl, Methylcyclohexenyl, Ethylcyclohexenyl,
— Cycloalkyl- oder Cycloalkenyl-alkyl, wobei die Cycloalkyl-, Cycloalkenyl- und Alkylgruppen wie oben definiert sind, z.B. Cyclopropylmethyl, Cyclopropylethyl, Cyclobutylmethyl, Cyclobutylethyl, Cyclopentylmethyl, Cyclopentylethyl, Cyclopentenylmethyl, Cyclopentenylethyl, Cyclohexylmethyl, Cyclohexylethyl, Cyclohexenylmethyl, Cyclohexenylethyl, bedeuten, dadurch gekennzeichnet, daß man ein 3,5-Dihalogenphenol der Formel V:

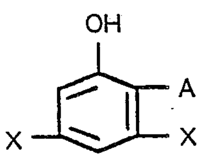

(V)

in der A und X wie in Anspruch 1 definiert sind, in Form eines Alkaliphenolates mit einem Amin der Formel VI:

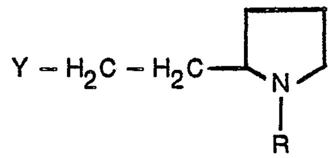

(VI)

,

in der Y ein anionischer, eliminierbarer Rest und R wie in Anspruch 1 definiert ist, umsetzt.

2. Verfahren nach Anspruch 1, dadurch gekennzeichnet, daß man die Phenoxyamine der allgemeinen Formel (I) mit einer Mineral- oder organischen Säure in Additionssalze mit einem Alkylsalz in quartäre Ammoniumsalze oder durch Oxidation in N-Oxide überführt.

3. Verfahren zur Herstellung optisch aktiver Formen der Verbindungen der Formel I nach Anspruch 1 oder 2, gekennzeichnet durch eine chemische Umsetzung mit einer optisch aktiven Säure.

4. Verfahren zur Herstellung von Verbindung nach einem der Ansprüche 1, 2 oder 3, gekennzeichnet durch die allgemeine Formel:

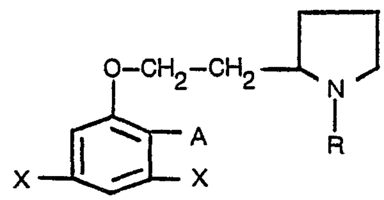

(II)

in der R, A und X wie oben definiert sind.

5. Verfahren zur Herstellung von Verbindungen nach einem der Ansprüche 1, 2 oder 3, gekennzeichnet durch die allgemeine Formel:

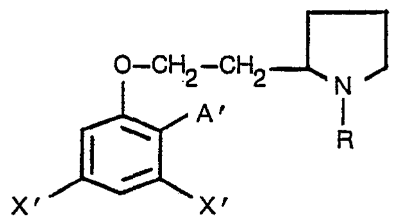

(III)

in der A′ H, $OCH_3$ oder $OC_2H_5$ sowie
X′ Cl oder Br bedeuten und
R wie oben definiert ist.

6. Verfahren zur Herstellung von 1-Ethyl-2-(2-methoxy-3,5-dichlorophenoxyethyl)-pyrrolidin-hydrochlorid gemäß einem der Ansprüche 1 bis 5.

7. Verfahren zur Herstellung von rechtsdrehendem 1-Ethyl-2-(2-methoxy-3,5-dichlorophenoxyethyl)-pyrrolidin hydrochlorid gemäß einem der Ansprüche 1 bis 5.

8. Verfahren zur Herstellung von linksdrehendem 1-Ethyl-2-(2-methoxy-3,5-dichlorophenoxyethyl)-pyrrolidin hydrochlorid gemäß einem der Ansprüche 1 bis 5.

9. Verfahren zur Herstellung von 1-Methyl-2-(2-methoxy-3,5-dichlorophenoxyethyl)-pyrrolidin-fumarat gemäß einem der Ansprüche 1 bis 5.

10. Verfahren zur Herstellung von rechtsdrehendem 1-Methyl-2-(2-methoxy-3,5-dichlorophenoxyethyl)-pyrrolidin gemäß einem der Ansprüche 1 bis 5.

11. Verfahren zur Herstellung von linksdrehendem 1-Methyl-2-(2-methoxy-3,5-dichlorophenoxyethyl)-pyrrolidin gemäß einem der Ansprüche 1 bis 5.

12. Verfahren zur Herstellung von 1-Allyl-2-(2-methoxy-3,5-dichlorophenoxyethyl)-pyrrolidin-fumarat gemäß einem der Ansprüche 1 bis 5.

13. Verfahren zur Herstellung von 1-Ethyl-2-(3,5-dichlorophenoxyethyl)-pyrrolidin gemäß einem der Ansprüche 1 bis 5.

14. Verfahren zur Herstellung von 1-Methyl-2-(2-methoxy-3,5-dibromophenoxyethyl)-pyrrolidin-fumarat gemäß einem der Ansprüche 1 bis 5.

15. Verfahren zur Herstellung von 1-Ethyl-2-(2-ethoxy-3,5-dichlorophenoxyethyl)-pyrrolidin-citrat gemäß einem der Ansprüche 1 bis 5.

16. Verfahren zur Herstellung von 1-Cyclohexyl-2-(2-methoxy-3,5-dichlorophenoxyethyl)-pyrrolidin-diphospat gemäß einem der Ansprüche 1 bis 5.

17. Verfahren zur Herstellung von 1-Cyclopropylmethyl-2-(2-methoxy-3,5-dichlorophenoxyethyl)-pyrrolidin-fumarat gemäß einem der Ansprüche 1 bis 5.

18. Verfahren zur Herstellung von 1-(1-Cyclohexenylmethyl)-2-[2-(2-methoxy-3,5-dichlorophenoxy)ethyl]-pyrrolidin-chlorhydrat gemäß einem der Ansprüche 1 bis 5.

**Claims for the Contracting States: BE CH DE FR GB IT LI LU NL SE**

1. Novel substituted heterocyclic phenoxyamines having the following general formula (I):

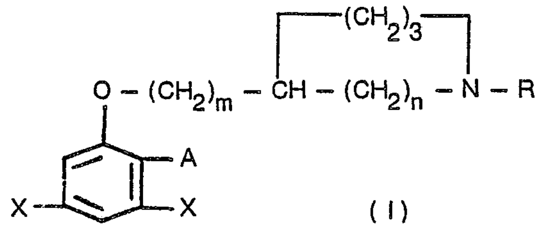

(I)

wherein m = 0 or 2
n = 0 or 2
provided that $m + n = 2$
and wherein:
A = — hydrogen
— alkoxy comprising from 1 to 4 carbon atoms such as methoxy or ethoxy, propoxy or butoxy, which is branched or straight-chained,
— alkenyloxy comprising from 2 to 6 carbon atoms such as vinyloxy, propenyloxy (allyloxy), butenyloxy, pentenyloxy, hexenyloxy,
X = — halogen such as F, Cl, Br,
R = — hydrogen
— lower alkyl comprising from 1 to 6 carbon atoms such as methyl or ethyl, propyl, butyl, pentyl or hexyl, which is branched or straight-chained,
— cycloalkyl comprising from 3 to 6 carbon atoms, which may be substituted by an alkyl group comprising 1 or 2 carbon atoms such as cyclopropyl, cyclobutyl, cyclopentyl, cyclohexyl, methylcyclobutyl, methylcyclopentyl, methylcyclohexyl, ethylcyclohexyl,
— alkenyl comprising from 3 to 6 carbon atoms such as prop-2-enyl (allyl), butenyl, pentenyl, hexenyl,
— cycloalkenyl comprising from 4 to 6 carbon atoms, which may be substituted by an alkyl group comprising 1 or 2 carbon atoms such as cyclobutenyl, cyclopentenyl, cyclohexenyl, methylcyclobutenyl, methylcyclopentenyl, methylcyclohexenyl, ethylcyclohexenyl,
— cycloalkyl- or cycloalkenyl-alkyl in which the cycloalkyl, cycloalkenyl and alkyl groups are as defined hereinbefore, such as cyclopropylmethyl, cyclopropylethyl, cyclobutylmethyl, cyclobutylethyl, cyclopentylmethyl, cyclopentylethyl, cyclopentenylmethyl, cyclopentenylethyl, cyclohexylmethyl, cyclohexylethyl, cyclohexenylmethyl and cyclohexenylethyl.

2. Novel compounds according to claim 1 characterised in that they are in the form of salts of addition with an organic or inorganic acid of quaternary ammonium salts or N-oxides.

3. A compound according to one of claims 1 or 2 characterised in that it is in the form of an optically active isomer.

4. A compound according to one of claims 1, 2 or 3 characterised by the following general formula:

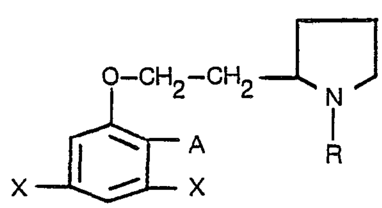

wherein
R, A and X are of the same definition as in those claims.

5. A compound according to one of claims 1, 2 or 3 characterised by the following general formula:

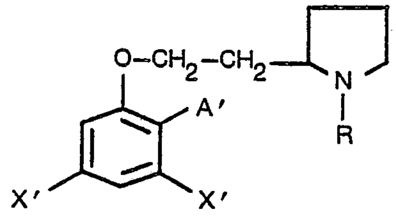

wherein:

A′ represents H, $OCH_3$, $OC_2H_5$
X′ represents Cl or Br
R is as defined hereinbefore.

6. According to one of claims 1 to 5, 1-ethyl-2-[2-methoxy 3,5-dichlorophenoxyethyl]pyrrolidine hydrochloride.

7. According to one of claims 1 to 5, dextrarotatory 1-ethyl-2-[2-methoxy 3,5-dichlorophenoxyethyl]pyrrolidine hydrochloride.

8. According to one of claims 1 to 5, levorotatory 1-ethyl-2-[2-methoxy 3,5-dichlorophenoxyethyl]pyrrolidine hydrochloride.

9. According to one of claims 1 to 5, 1-methyl-2-[2-methoxy 3,5-dichlorophenoxyethyl]pyrrolidine fumarate.

10. According to one of claims 1 to 5, dextrorotatory 1-methyl-2-[2-methoxy 3,5-dichlorophenoxyethyl]pyrrolidine.

11. According to one of claims 1 to 5, levorotatory 1-methyl-2-[2-methoxy 3,5-dichlorophenoxyethyl]pyrrolidine.

12. According to one of claims 1 to 5, 1-allyl-2-[2-methoxy 3,5-dichlorophenoxyethyl]pyrrolidine fumarate.

13. According to one of claims 1 to 5, 1-ethyl-2-[3,5-dichlorophenoxyethyl]pyrrolidine.

14. According to one of claims 1 to 5, 1-methyl-2-[2-methoxy 3,5-dibromophenoxyethyl]pyrrolidine fumarate.

15. According to one of claims 1 to 5, 1-ethyl-2-[2-ethoxy 3,5-dichlorophenoxyethyl]pyrrolidine citrate.

16. According to one of claims 1 to 5, 1-cyclohexyl-2-[2-methoxy-3,5-dichlorophenoxyethyl]pyrrolidine bis phosphate.

17. According to one of claims 1 to 5, 1-cyclopropyl-methyl-2-[2-methoxy, 3,5-dichlorophenoxyethyl]pyrrolidine fumarate.

18. According to one of claims 1 to 5, 1-(1-cyclohexenylmethyl)-2-[2-(2-methoxy-3,5-dichlorophenoxy)ethyl]pyrrolidine hydrochloride.

19. According to one of claims 6, 7, 8, 9, 12, 14, 15, 16, 17, 18, phenoxyamine freed of its salt.

20. As novel medicaments, having a local anaesthetic activity, the compounds according to claims 1 to 19.

21. Pharmaceutical compositions characterised in that they contain as their active ingredient one of the compounds according to claims 1 to 19, combined with pharmaceutically acceptable excipients.

22. A process for preparing the compounds according to claims 1 to 18, which comprises treating a 3,5-dihalogenated phenol having the following formula V:

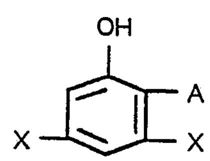

(V)

wherein

A and X are as defined in claim 1, in the form of alkaline phenolate with an amine of formula VI:

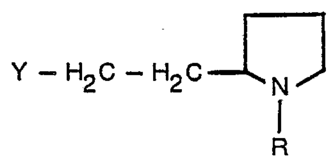

(VI)

wherein

Y is an anionic residue which can be eliminated and R is as defined in claim 1.

23. A process for producing optically active forms of the compounds of formula I, by chemical combination with an optically active acid.

**Claims for the Contracting State: AT**

1. Method for preparing heterocyclic substituted phenoxyamines with the general formula (I):

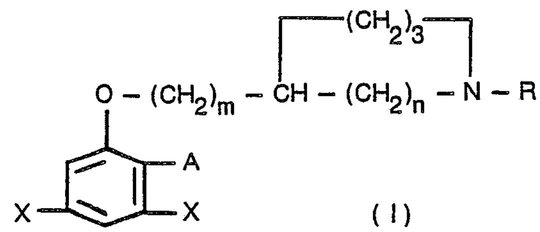

(I)

in which m = 0 or 2

n = 0 or 2

under the condition that m + n = 2,

and in which

A = — hydrogen,

— alkoxy having 1—4 carbon atoms, such as methoxy or ethoxy, linear or branched propoxy or butoxy,

— alkenyloxy having 2—6 carbon atoms, such as vinyloxy, propenyloxy (allyloxy), butenyloxy, pentenyloxy, hexenyloxy,

X = — halogen, such as F, Cl, Br,

R = — hydrogen,

— lower alkyl with 1—6 carbon atoms such as methyl or ethyl, linear of branched propyl, butyl, pentyl or hexyl,

— cycloalkyl with 3—6 carbon atoms, which is optionally substituted by an alkyl group with 1 or 2 carbon atoms, such as cyclopropyl, cyclobutyl, cyclopentyl, cyclohexyl, methylcyclobutyl, methylcyclopentyl, methylcyclohexyl, ethylcyclohexyl,

— alkenyl with 3—6 carbon atoms, such as propen-2-yl (allyl), butenyl, pentenyl, hexenyl,

— cycloalkenyl with 4—6 carbon atoms which is optionally substituted by an alkyl group with 1—2 carbon atoms, such as cyclobutenyl, cyclopentenyl, cyclohexenyl, methylcyclobutenyl, methylcyclopentenyl, methylcyclohexenyl, ethylcyclohexenyl,

— cycloalkyl — or cycloalkenyl-alkyl in which cycloalkyl, cycloalkenyl and alkyl groups are defined as above, such as cyclopropylmethyl, cyclopropylethyl, cyclobutylmethyl, cyclobutylethyl, cyclopentylmethyl, cyclopentylethyl, cyclopentenylmethyl, cyclopentenylethyl, cyclohexylmethyl, cyclohexylethyl, cyclohexenylmethyl, cyclohexenylethyl by reacting a 3,5-dihalogenated phenol with the formula:

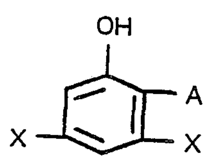

in which A and X are defined as above, in form of an alkali phenolate with an amine of the formula:

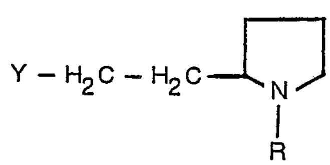

in which Y is an anionic group which can be eliminated and R is defined as above.

2. Method for preparation according to claim 1, characterized in that the phenoxyamines of formula (I) are transformed into addition salts by means of a mineral or organic acid, into quarternary ammonium salts by means of an alkylate (alkyl salt) or oxidized to the N-oxides.

3. Method for preparation according to claims 1 or 2, characterized in that optically active forms of compounds of formula (I) are obtained by chemical combination with an optically active acid.

4. Method for preparation according to claims 1, 2 or 3, of compounds with the general formula (I) having the structure (II)

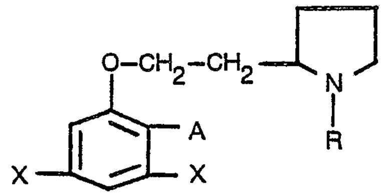

(II)

in whch R, A and X have the same definitions as in these claims.

5. Method for preparation according to claims 1, 2 or 3 of compounds with the general formula (I) having the structure (III)

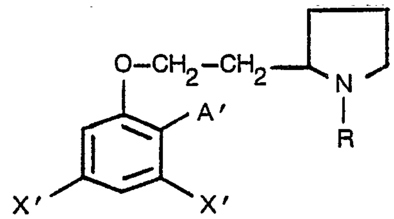

(III)

in which

A′ is H, $OCH_3$, $OC_2H_5$,

X is Cl or Br, and

R is defined as above.

6. According to one of the claims 1, 2, 4 and 5, a method for preparing the chlorohydrate of 1-ethyl-2-[2-methoxy-3,5-dichlorophenoxyethyl]-pyrrolidine.

7. According to one of the claims 1—5, a method for preparing the chlorhydrate of dextrorotatory 1-ethyl-2-[2-methoxy-3,5-dichlorophenoxyethyl]pyrrolidine.

8. According to one of the claims 1—5, a method for preparing the chlorohydrate of levorotatory 1-ethyl-2-[2-methoxy-3,5-dichlorophenoxyethyl]-pyrrolidine.

9. According to one of the claims 1, 2, 4, or 5, a method for preparing the fumarate of 1-methyl-2-[2-methoxy-3,5-dichlorophenoxyethyl]-pyrrolidine.

10. According to one of the claims 1—5, a method for preparing dextrorotatory 1-methyl-2-[2-methoxy-3,5-dichlorophenoxyethyl]-pyrrolidine.

11. According to one of the claims 1—5, a method for preparing levorotatory 1-methyl-2-[2-methoxy-3,5-dichlorophenoxyethyl]-pyrrolidine.

12. According to one of the claims 1, 2, 4 or 5, a method for preparing the fumarate of 1-allyl-2-[2-methoxy-3,5-dichlorophenoxyethyl]-pyrrolidine.

13. According to one of the claims 1, 2, 4 or 5, a method for preparing 1-ethyl-2-[3,5-dichlorophenoxyethyl]-pyrrolidine.

14. According to one of the claims 1, 2, 4 or 5, a method for preparing the fumarate of 1-methyl-2-[2-methoxy-3,5-dibromophenoxyethyl]pyrrolidine.

15. According to one of the claims 1, 2, 4 or 5, a method for preparing the citrate of 1-ethyl-2-[2-ethoxy-3,5-dichlorophenoxyethyl]-pyrrolidine.

16. According to one of the claims 1, 2, 4 or 5, a method for preparing the bis-phosphate of 1-cyclohexyl-2-[2-methoxy-3,5-dichlorophenoxyethyl]-pyrrolidine.

17. According to one of the claims 1, 2, 4 or 5, a method for preparing the fumarate of 1-cyclopropylmethyl-2-[2-methoxy-3,5-dichlorophenoxyethyl]-pyrrolidine.

18. According to one of the claims 1, 2, 4 or 5, a method of preparing the chlorohydrate of 1-(1-cyclohexenylmethyl)-2-[2-(2-methoxy-3,5-dichlorophenoxy)ethyl]-pyrrolidine.